# EUROPEAN PATENT APPLICATION

(11) **EP 2 017 267 A1**
(43) Date of publication of application: **21.01.2009**
(21) Application number: 07112529.8
(22) Date of filing: 16.07.2007
(51) Int. Cl.: C07D 239/42, C07D 309/10, C07C 217/46

(54) **Synthesis of statins**

(71) Applicant: LEK Pharmaceuticals d.d., 1526 Ljubljana (SI)
(72) Inventor: Kopitar, gregor, 1235 Radomlje, (SI); Mrak, Peter, 4209 Zabnica (SI); Oslaj, Matej, 4000 Kranj (SI)
(74) Representative: Kröger, Bernd

(57) **Abstract**

The process for synthesis of statins is presented where the intermediate intermediate (4*R*,6*S*)-6-(dialkoxymethyl)tetrahydro-2*H*-pyran-2,4-diol which already possesses the desired stereochemistry corresponding to final statin is prepared by an aldolaze.

## Description

### Field of the Invention

The present invention relates in general to the field of biotechnology and in particular to a process for the preparation of intermediates for HMG-CoA reductase inhibitors, known also as statins, particularly to rosuvastatin. Specifically this invention relates to preparation of a common intermediate which can be used for preparation of all statins.

### Background of the Invention

Statins, of which the representative examples may be selected from rosuvastatin, cerivastatin, atorvastatin, fluvastatin, pitavastatin, bervastatin, dalvastatin or their analogs or pravastatin, simvastatin, lovastatin or their analogs share a characteristic structure, consisting of respectively a heptenoic or heptanoic acid moiety (free acid, salt or lactone) connected to the aromatic or alicyclic core. Biological activity of statins is closely related to their stereochemistry, especially configuration at the chiral atoms of said heptenoic or heptanoic acid moiety.

The sequential aldol reactions catalyzed by deoxyribose aldolase are described in J. Am. Chem. Soc.; 117, 29, (1995) pp 7585. Use of aldolases (in particular 2-deoxyribose-5-phosphate aldolase) in the synthesis of statins is known from WO 06/134482. Enzymed used belong to class: EC 4.1.2.4. - deoxyribose -5-phosphate aldolaze. WO 05/118794 is concerned with improving the enzyme using known substrates.

### Disclosure of the Invention

Our invention is concerned with choice of suitable substrates which are enyzmaticaly converted into (4R,6S)-6-(dialkoxymethyl)tetrahydro-2H-pyran-2,4-dioles or (4R,6S)-6-(alkylenedioxy)tetrahydro-2*H*-pyran-2,4-dioles.

One aspect of the invention is a process comprising the step of reacting a substrate (I) with an acetaldehyde under aldolase-catalysed aldol condensation conditions to form the corresponding lactol (III) 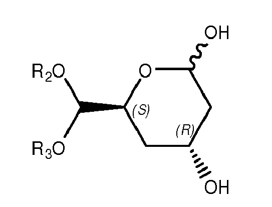 wherein the substrate (I) has formula 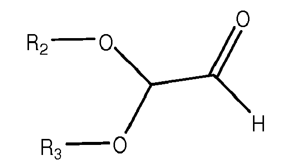 where R₂ and R₃ are independently selected from C1 - C4 alkyl, or together form a cyclic structure of formula (CH₂)ₙ where n is from 2 to 6.

Another aspect of the invention is a process for manufacturing rosuvastatin or a pharmaceuticaly acceptable salt thereof comprising the steps of
a) reacting a substrate (I) with an acetaldehyde under aldolase-catalysed aldol condensation to form the corresponding lactol (III) wherein the substrate (I) has formula 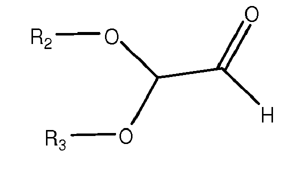 where R₂ and R₃ are independently selected from C1 - C4 alkyl, or together form a cyclic structure of formula (CH₂)ₙ where n is from 2 to 6;
b) converting said lactol into compound of formula 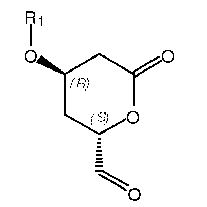
c) subjecting said compound obtained in step b) to conditions sufficient to produce rosuvastatin or a pharmaceuticaly acceptable salt thereof.

The particular aspects of the invention are where acceptor substrate is selected among: 2,2-dimethoxyethanal (preferred), 2,2-diethoxyethanal, 2,2-dipropoxyethanal, 2,2-dibutoxyethanal, 1,3-dioxolane-2-carbaldehyde, 4-methyl-1,3-dioxolane-2-carbaldehyde" 4-ethyl-1,3-dioxolane-2-carbaldehyde" 4-propyl-1,3-dioxolane-2-carbaldehyde,, 4-butyl-1,3-dioxolane-2-carbaldehyde, 4,5-dimethyl-1,3-dioxolane-2-carbaldehyde and a process wherein said aldolase is selected from DERA 01, DERA 02, DERA 03, DERA 04, DERA 05, DERA 06, DERA 07, DERA 08, DERA 09, DERA 10, DERA 11, DERA 12 DERA 13, DERA 14, DERA 15, DERA 16, DERA 17, DERA 18, DERA 19, DERA 20, DERA 21 DERA 22, nd DERA 23 or an aldolase having an amino acid sequence identity of at least about 70% to amino acid sequence of any of said aldolases, and in particular wherein said aldolase has an amino acid sequence identity of at least about 70% to amino acid sequence of SEQ ID NO: 2 or wherein said aldolase has an amino acid sequence identity of at least about 90% to amino acid sequence of SEQ ID NO: 5 or wherein said aldolase has an amino acid sequence identity of at least about 80% to amino acid sequence of SEQ ID NO: 17.

The process aspect of invention are embodied in reaction conditions wherein pH for aldolase-catalysed aldol condensation is maintained in range 6 to 10, in particular wherein pH is maintained with buffer in pH range 7 to 9, more particular in use of phosphate buffer.

Another aspect of the invention is use of aldolase for the reaction of a substrate (I) with an acetaldehyde under aldolase-catalysed aldol condensation conditions to form the corresponding lactol (III) wherein the substrate (I) has formula 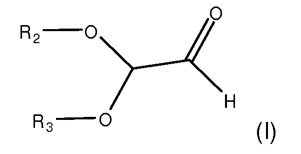 where R₂ and R₃ are independently selected from C1 - C4 alkyl, or together form a cyclic structure of formula (CH₂)ₙ where n is from 2 to 6 in particular wherein said aldolase is 2-deoxyribose-5-phosphate aldolase, more in particular wherein aldolase is selected from DERA 01 to DERA 23. In particular said aldolase is in aspect of the invention comprised within living whole cell, or is comprised within inactivated whole cell, or is comprised within homogenized whole cell, or is comprised within cell free extract, or is purified enzyme, or is immobilized, or is in form of extracelularly expressed protein.

Invention is in an aspect a process for preparing the compound of formula (III) 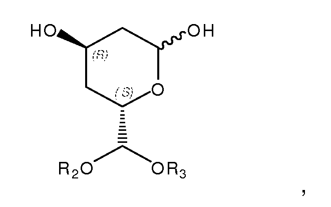 wherein R₂ and R₃ are independently selected from C1 - C4 alkyl, or together form a cyclic structure of formula (CH₂)ₙ where n is from 2 to 6,
consisting of steps of:
a) admixing the substrate of formula (I) 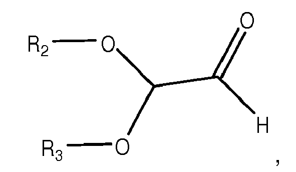 wherein R₂ and R₃ are independently selected from C1 - C4 alkyl, or together form a cyclic structure of formula (CH₂)ₙ where n is from 2 to 6,
   with
   acetaldehyde and
   2-deoxyribose-5-phosphate aldolase
   in an aqueous medium at pH value of 7 to 9, specifically 7,1 to 8,5 at temperature at about 30 - 50° Cm specificlay 30 to 40 °C to form a reaction mixture;
b) maintaining said reaction mixture at said temperature and pH value for time period between 1 h and 6 h; and
c) recovering said compound of formula (III),

Aspect of the invention are novel compounds of formula: 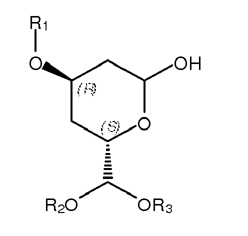 where R₁ is H or a protecting group; and R₂ and R₃ are independently selected from C1 - C4 alkyl, or together form a cyclic structure of formula (CH₂)ₙ where n is from 2 to 6.

### Detailed description of the Invention

### T

The present invention provides for enzymatic synthesis of intermediates **AVL',** which is chemically (4*R*,6*S*)-6-(dialkoxymethyl)tetrahydro-2*H*-pyran-2,4-diol or (4*R*,6*S*)-6-(alkylenedioxy)tetrahydro-2H-pyran-2,4-diol of general formula: 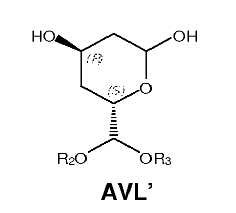 where R₂ and R₃ are independently selected from C1 - C4 alkyl, or together form (an optionally C1-C4 alkyl substituted) cyclic structure of formula (CH₂)ₙ where n is from 2 to 6.

The feature of an early intermediate **AVL'** and in particular **AVL,** which is chemically (4*R*,6*S*)-6-(dimethoxymethyl)tetrahydro-2*H*-pyran-2,4-diol is that is possesses the desired stereochemistry, avoiding subsequent separations of later intermediates. **AVL'** or **AVL** is chemically an aldehyde acetal which behaves as a masked (protected) aldehyde what eliminates the necessity for the oxidation synthetic step in the synthesis of statins. Rosuvastatin can be prepared starting from said early intermediate **AVL'** as disclosed on following scheme.

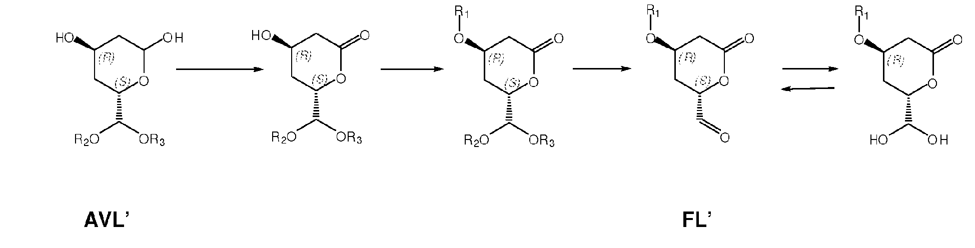

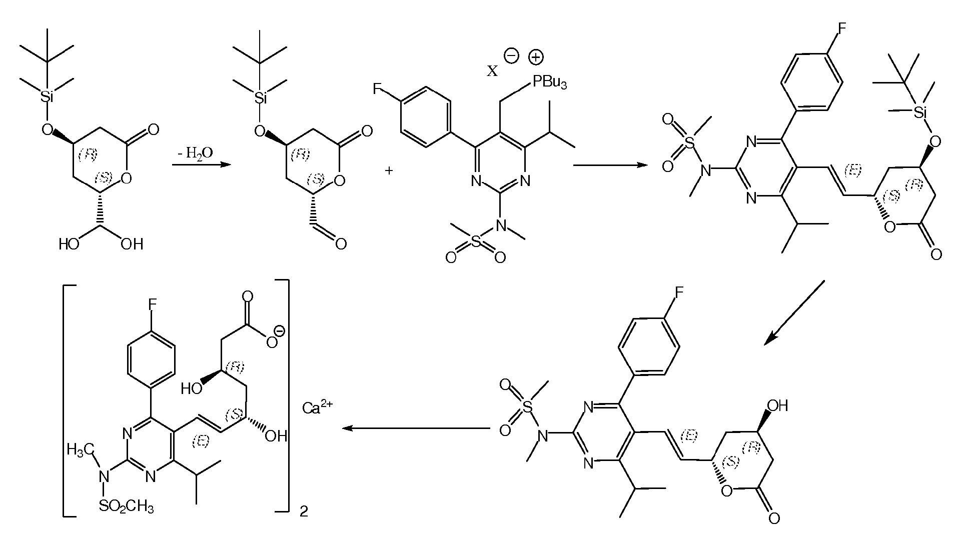

To produce other statins the (2*S*,4*R*)-4-(*protected*)-6-oxo-tetrahydro-2*H*-pyran-2-carbaldehyde **FL'** or its hydrate should be reacted under condition of Wittig coupling with an appropriate reagent followed by hydrogenation when needed.

The appropriate reagent is a heterocylic or alicyclic derivatives (skeleton of statin) of following formula : 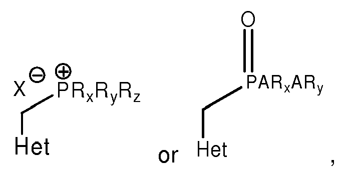 where A can be a bond or O;
and wherein Rₓ, R_{y}, and R_{z}, are the same or different and are selected from optionally substituted C₁ - C₈ alkyl or C₃-C₆ cycloalkyl or C₁ - C₈ alkenyl or C₅-C₆ cycloalkenyl or aryl;
and X is an anion, preferably halogen or RCOO⁻ anion, more preferably chloride, bromide or trifluoroacetate;
and Het is selected so that it forms a heterocyclic or alicyclic skeleton of a statin;
other HMG-CoA reductase inhibitors (preferably selected among cerivastatin, fluvastatin, pitavastatin, bervastatin, dalvastatin) can be analogously prepared.

Heterocyclic or alicyclic skeleton of statin is in particular selected from:

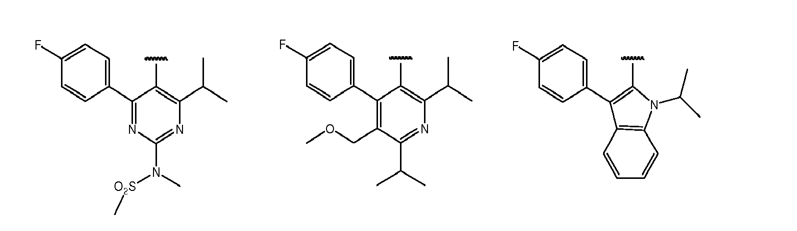

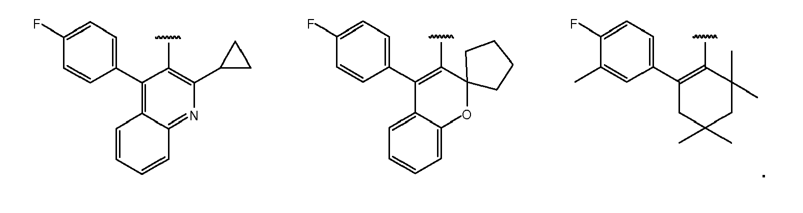

The invention provides enzymatic process using the substrate (I) and acetaldehyde (II) to form corresponding lactole (III) in an aldolase catalysed aldol condensation reaction as presented on following scheme: 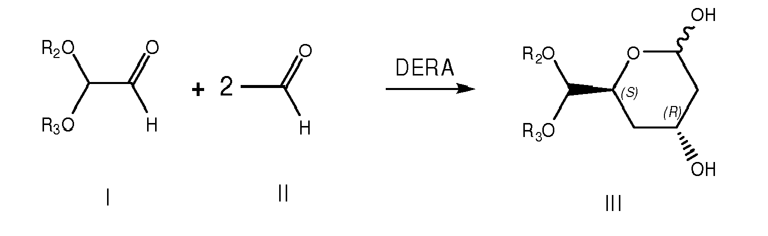 where R₂ and R₃ are independently selected from C1 - C4 alkyl, or together form an optionally substituted cyclic structure of formula (CH₂)ₙ where n is from 2 to 6. Structure III according to the invention has a strictly defined stereoisomery at position 4 and 6, while other chiral centers may be present in both possibilities forming mixtures of epimers. The changeable chiral centers are not essential for the synthesis of the final product of the invention because the chirality of these carbon atoms is lost during the synthetic procedure.

The term "aldolase-catalyzed aldol condensation conditions" as used herein refers to any aldol condensation conditions known in the art that can be catalyzed by an aldolase, as described herein. In particular the aldolase-catalysed aldol condensation conditions are such that allow forming and accumulation of desired product. These conditions include in one aspect that the aldolase is active enzyme provided at sufficient load to be able to perform the sequential condensation, in another aspect that the substrate and acetaldehyde are present in the reaction in an amount that does not inhibit the activity of the aldolase, in another aspect that the temperature, pH, solvent composition, agitation and length of reaction allow accumulation of desired product, in another aspect that said conditions do not have detrimental effect on product stability. Specifically those conditions are defined by values disclosed in examples.

Aldolase activity towards above substrate (I) means that specified enzyme either isolated and or purified, or immobilized or within living cell, or comprised within inactivated whole cell, or comprised in homogenized cell material, or in cell free extract will catalyze the above reaction giving of I and II giving III.

The term "conditions sufficient to produce statin (in particular rosuvastatin) or a pharmaceutically acceptable salt thereof" as used herein refers to those means described in the art, including those means described herein.

The term an "organism over expressing biologically active form of an aldolase" as used herein refers to any organism having the aldolase expression under control of a strong promoter, and where the aldolase is expressed at high levels (compared to w.t. expression control) and is accumulated intracellularly of extracellularly. The process of making such organism is well known to a person skilled in the art. The present invention provides an example of making such organism.

An aldolase for use in the present invention may be any compound that has aldolase activity towards above substrate (I) In one embodiment of the invention, the aldolase is a 2-deoxyribose-5-phosphate aldolase (DERA). Examples of a suitable DERA - aldolase include, but are not limited to: DERA 01, DERA 02, DERA 03, DERA 04, DERA 05, DERA 06, DERA 07, DERA 08, DERA 09, DERA 10, DERA 11, DERA 12, DERA 13, DERA 14, DERA 15, DERA 16, DERA 17, DERA 18, DERA 19, DERA 20, DERA 21,DERA 22 and DERA 23 which are identified by their nucleotide sequences or respective codon optimized nucleotide sequences or amino acid sequences set forth in sequence listings.

In general any of the DERA aldolases known in art may be used for the reaction regardless of their sequence identity to the above listed DERA aldolases. The invention provides examples of performing said reactions successfully with two different aldolases having only 30.1% identity. The yields of the reaction however may depend on each aldolases substrate specificity and inhibitory effects of the substrates on each aldolase.
DERA 01 is an aldolase having a nucleotide sequence of SEQ ID NO: 1 or an amino acid sequence of SEQ ID NO: 2; DERA 01 (E. Coli) is commercially available from Sigma Aldrich, St. Louis, MO, USA, under catalog number 91252.
DERA 02 is an aldolase having a nucleotide sequence of SEQ ID NO: 3 or SEQ ID NO: 4 or an amino acid sequence of SEQ ID NO: 5; DERA 02 is described in William A. Greenberg, et al., PNAS, (2004), Vol. 101 , No. 16, pp. 5788
DERA 03 is an aldolase having a nucleotide sequence of SEQ ID NO: 6 or an amino acid sequence of SEQ ID NO: 7
DERA 04 is an aldolase having a nucleotide sequence of SEQ ID NO: 8 or an amino acid sequence of SEQ ID NO: 9
DERA 05 is an aldolase having a nucleotide sequence of SEQ ID NO: 10 or an amino acid sequence of SEQ ID NO: 11
DERA 06 is an aldolase having a nucleotide sequence of SEQ ID NO: 12 or an amino acid sequence of SEQ ID NO: 13
DERA 07 is an aldolase having a nucleotide sequence of SEQ ID NO: 14 or an amino acid sequence of SEQ ID NO: 15
DERA 08 is an aldolase having a nucleotide sequence of SEQ ID NO: 16 or an amino acid sequence of SEQ ID NO: 17
DERA 09 is an aldolase having a nucleotide sequence of SEQ ID NO: 18 or an amino acid sequence of SEQ ID NO: 19
DERA 10 is an aldolase having a nucleotide sequence of SEQ ID NO: 20 or an amino acid sequence of SEQ ID NO: 21
DERA 11 is an aldolase having a nucleotide sequence of SEQ ID NO: 22 or an amino acid sequence of SEQ ID NO: 23
DERA 12 is an aldolase having a nucleotide sequence of SEQ ID NO: 24 or an amino acid sequence of SEQ ID NO: 25
DERA 13 is an aldolase having a nucleotide sequence of SEQ ID NO: 26 or an amino acid sequence of SEQ ID NO: 27
DERA 14 is an aldolase having a nucleotide sequence of SEQ ID NO: 28 or an amino acid sequence of SEQ ID NO: 29
DERA 15 is an aldolase having a nucleotide sequence of SEQ ID NO: 30 or an amino acid sequence of SEQ ID NO: 31
DERA 16 is an aldolase having a nucleotide sequence of SEQ ID NO: 32 or an amino acid sequence of SEQ ID NO: 33
DERA 17 is an aldolase having a nucleotide sequence of SEQ ID NO: 34 or an amino acid sequence of SEQ ID NO: 35
DERA 18 is an aldolase having a nucleotide sequence of SEQ ID NO: 36 or an amino acid sequence of SEQ ID NO: 37
DERA 19 is an aldolase having a nucleotide sequence of SEQ ID NO: 38 or an amino acid sequence of SEQ ID NO: 39
DERA 20 is an aldolase having a nucleotide sequence of SEQ ID NO: 40 or an amino acid sequence of SEQ ID NO: 41
DERA 21 is an aldolase having a nucleotide sequence of SEQ ID NO: 42 or an amino acid sequence of SEQ ID NO: 43
DERA 22 is an aldolase having a nucleotide sequence of SEQ ID NO: 44 or an amino acid sequence of SEQ ID NO: 45
DERA 23 is an aldolase having a nucleotide sequence of SEQ ID NO: 46 or an amino acid sequence of SEQ ID NO: 47

The aldolase encompases aldolase having an amino acid sequence identity of at least about 50% thereof; preferably, at least 70% thereof, to a aldolases described herein. The amino acid sequence identities are determined by analysis with sequence comparison algorithm or by visual inspection. In one aspect, the sequence comparison algorithm is made with AlignX algorithm of Vector NTI 9.0 ( InforMax ) with settings set to default.

In particular the invention provides for a process comprising the step of reacting a substrate (I) with an acetaldehyde under aldolase-catalysed aldol condensation conditions to form the corresponding lactol (III) 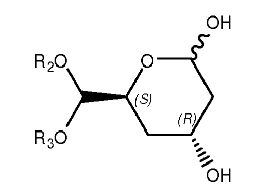 wherein the substrate (I) has formula 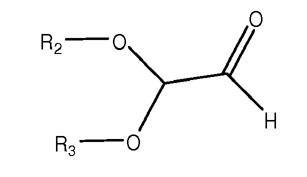 where R2 and R3 are independently selected from C1 - C4 alkyl, or together form a cyclic structure of formula (CH2)n where n is from 2 to 6, and subsequent use thereof in the synthesis of statins (in particular rosuvastain); wherein the aldolaze is selected in first embodiment from DERA 01 or DERA 06 or DERA 07, or any aldolase having an amino acid sequence identity of at least about 90% to those or in another embodiment wherein the aldolaze is selected in first embodiment from DERA 02 or DERA 15 or DERA 16, or any aldolase having an amino acid sequence identity of at least about 90% to those.

The DERA aldolases described herein can be prepared by any means known in the art, including but not limited to standard protocols for protein expression in recombinant E. coli such as described in Sambrook and Russell, Molecular Cloning: A Laboratory Manual, 3'^" Ed., Cold Spring Harbor, NY 2001. Modified versions of known DERA aldolases may be necessary or may result depending on cloning conditions and are encompassed by the present invention.

The DERA aldolases described herein can be used in any biologically active form. In one embodiment the aldolase is active and can be used in the form of living whole cell catalyst. In one embodiment the aldolase is active and can be used in the form of inactivated whole cell catalyst. In one embodiment the the aldolase is active and can be used in the form of homogenized whole cell catalyst. In one embodiment the the aldolase is active and can be used in the form of cell free extract. In one embodiment the the aldolase is active and can be used in form purified enzyme by means of any methods known in the art. In another aspect the aldolase is active and can be used in form of extracelularly expressed protein.

Alternatively the invention provides a method of making intermediates for production of statins by using whole cell catalyst. The whole cell catalyst in one embodiment is any prokaryotic organism over expressing biologically active form of an aldolase. In particular embodiment the whole cell catalyst is Escherichia coli over expressing biologically active form of an aldolase.

Substrates and reaction conditions were chosen to give optimum activity of an aldolase used to make the intermediates useful for statin production.

The substrates (I) are in first aspect selected according to their inhibitory effects towards aldolase activity. In particular the acceptor substrates with the least inhibitory effect are suitable for the reaction.

The substrates (I) are also selected according to their and to the corresponding lactol (III) product stability at optimal reaction conditions. In particular the acceptor substrates with the best stability are preferred for the reaction.

The substrates (I) may be in particular 2,2-dimethoxyethanal (preferred), 2,2-diethoxyethanal, 2,2-dipropoxyethanal, 2,2-dibutoxyethanal, 1,3-dioxolane-2-carbaldehyde, 4-methyl-1,3-dioxolane-2-carbaldehyde" 4-ethyl-1,3-dioxolane-2-carbaldehyde" 4-propyl-1,3-dioxolane-2-carbaldehyde" 4-butyl-1,3-dioxolane-2-carbaldehyde, 4,5-dimethyl-1,3-dioxolane-2-carbaldehyde,. Alternatively the substrates may be the structures which in conditions of the enzymatic reaction lead to said abstracts in situ like 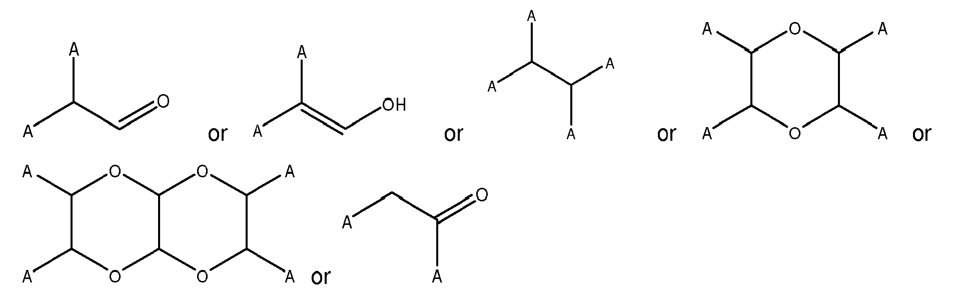 wherein substituents A are the same or different selected from OH, C1-C4 alkoxy, halo and azido groups.

Generally aldolase will be provided in a suitable vessel or reactor, and the substrate (I) and acetaldehyde will be added batch-wise or continuously.

Specifically aldolase is prepared in an aqueous solution (particularly in concentration 0,1 g/L to 3g/L) optionally in presence of salt (in particular NaCl in concentration from 50 to 500 mM), aqueous solution may contain organic solvents miscible with water( in particular dimethyl sulfoxide in concentration from 2 to 15% V/V), and may be buffered to pH 6 to 11. Suitable buffers can be prepared from: acids, bases, salts or mixtures thereof, and any other buffer system known in the art except those possessing primary, secondary or tertiary amino group. In particular, phosphate buffer, in concentration 10 to 500 mM can be used. The aqueous solution can also be prepared by adding the said aldolase to water and maintaining pH during the reaction by means of automated addition of inorganic acids, bases, salts or mixtures thereof.

In the process aspect the substrate (I) may be added to the reaction mixture continuously or alternatively the substrate (I) are added to the reaction mixture in one batch or more batches. In one aspect the total amount of substrates added to the mixture is such that the total amount of substate (I) added would be from about 20 mmol per liter of reaction mixture to about 2 mol per liter of reaction mixture, in particular from about 100 mmol per liter of reaction mixture to about 1,5 mmol per liter of reaction mixture, more particular from about 200 mmol per liter of reaction mixture to about 700 mmol per liter of reaction mixture. Acetaldehyde may be added by several means. In one aspect the acetaldehyde is added to the reaction mixture in one batch or more batches or alternatively continuously. Acetaldehyde may be premised with substrate (I) and added to reaction mixture. The total amount of acetaldehyde added to the reaction mixture is from about 0.1 to about 4 molar equivalents to total amount of acceptor substrate, in particular from about 2 to about 2,5 molar equivalents.

Alternatively aldolase may be added to reaction mixture containing at least one of substrate (I) or acetaldehyde. The reaction mixture is understood to comprise solvent and at least one of aldolase or substrate (I) or acetaldehyde.

In one aspect the pH used for aldolase-catalyzed reaction is from about pH 5 to about pH 12. In one embodiment the pH used for aldolase-catalyzed reaction is from about pH 6 to about pH 10. In one embodiment the pH used for aldolase-catalyzed reaction is from about pH 7 to about pH 9. Specifically the pH will be mantaind by suitable buffer in range 7,2 to 8,5.

Some commonly used buffers can lover the yield of the forementioned aldolase-catalysed reaction by limiting availability of aldolase-condensation intermediates particularly first condensation reaction products as they may undergo chemical reaction with a buffer. We discovered that bis-tris propan reacts with said intermediates ( (S)-3-hydroxy-4,4-dimethoxybutanal ) giving (*S,Z*)-2-(3-((1,3-dihydroxy-2-(hydroxymethyl)propan-2-yl)(3-hydroxy-4,4-dimethoxybut-1-enyl)amino)propylamino)-2-(hydroxymethyl)propane-1,3-diol. Other buffers that may react similarily are bis-tris, tricin, tris, bicin or any other buffer having primary, secondary or tertiary amino group. Thus a suitable buffers for adjusting the pH, if this adjustment is needed, are made with acids, bases, salts or mixtures thereof in particular phosphoric acid and sodium hydroxyde.

In one aspect the temperature used for aldolase-catalyzed reaction is from about 20 to about 70 °C. In one embodiment the temperature used for aldolase-catalyzed reaction is from about 25 to about 60 °C. In one embodiment the temperature used for aldolase-catalyzed reaction is from about 30 to about 50 °C.

The reaction is industrially suitable, as it proceeds to completion within few hours.

The effects of reaction conditions, notably pH, temperature and reaction time are surprising, especially in view of teaching of J. Am. Chem. Soc.; 117, 29, (1995) pp 7585, where the reaction with 2,2-dimethoxyethanal did not proceed even in 6 days.

After completion of reaction the enzyme is removed from the reaction mixture by addition of at least about 1 vol. of acetonitrile to 1 vol of reaction mixture. Alternatively the enzyme is removed by any salting out method known in the art. In one embodiment the salting out is performed with addition of ammonium sulfate of at least 5% m/V.

The invention also provides purification method for obtaining pure lactols produced by reaction. In one aspect the acetonitrile is evaporated from the reaction mixture and aqueous solution remaining is than liophylised. In another aspect the salting-out solution is liophylised. The powdered remain is than suspended in MTBE (methyl-t-butylether) /methanol 1:1. The suspension is filtered to remove unsoluble salts and fitrate is loaded to a silicagel column using MTBE/Methanol 1:1 as mobile phase.

In particular embodiment the invention provides for the reaction of 2,2-dimethoxyethanal with acetaldehyde under aldolase-catalysed aldol condensation to form (4R,6S)-6-(dimethoxymethyl)-tetrahydro-2H-pyran-2,4-diol wherein the used aldolase is DERA 01, DERA 02 or DERA... in an appropriate solvent (in particular aqueous solvent, which may be water in mixture with water soluble organic solvent) in ph range from 6 to 11, in particular 7 to 9 (adjusted if needed with acids, bases, salts or mixtures thereof in particular with phosphoric acid and sodium hydroxyde), wherein the reaction proceeds at temperature around 35-40 ° C and the conversion is finished in 1 to 6 hours.

In general aldolaze used is prepared by methods of protein expression described in in Sambrook, et al. (1989) Molecular cloning: A laboratory Manual 2nd Edition, New York: Cold Spring Harbor Laboratory Press, Cold Spring Harbor. Gene coding aldolase is cloned into an expression vector and the enzyme is expressed in suitable expression host.

The reaction yields are calculated relatively to total amount of substrate (I) added to the reaction mixtures and they are determined as ratio between moles of isolated product and moles of substrate (I) added to the reaction mixture.

The following examples illustrate the process of the present invention and are not intended to limit the scope of the invention.

### Example 1

### Preparation of aldolase

Escherichia coli gene deoC has been amplified using oligonucleotide primers CGGGATCCACTGATCTGAAAGCAAGCAGCC (SEQ ID NO: 48) and GCAAGCTTGCTGCTGGCGCTCTTACC (SEQ ID NO: 49) in a PCR reaction using isolated genome DNA from E. coli K-12 strain. The product was cleaved with restriction endonucleases BamHI and HindIII and the resulting fragment has been separated on agarose gel electrophoresis and purified. An expression vector pQE30 (Qiagene inc., Valencia, CA, USA) has been cleaved using the same fore mentioned restriction endonucleases and purified. The fragments have been assembled in a T4 ligase reaction. Competent Escherichia coli DH5alpha cells were transformed with the above mentioned ligation reaction. Ampicilin resistance colonies were cultured and plasmid DNA has been isolated. The resulting construct has been designated pQE30DeraC and sequenced for conformation of the gene sequence. Aldolase expressing organism has been prepared by transforming competent Escherichia coli TOP10 F' strain (Invitrogen corp., Carlsbad, CA, USA) with vector pQE30DeraC. The methods used for the process are described in Sambrook, et al. (1989) Molecular cloning: A laboratory Manual 2nd Edition, New York: Cold Spring Harbor Laboratory Press, Cold Spring Harbor and are well known to a person skilled in the art.

Terrific Broth media (150 mL, 12 g/L bacto tryptone, 24 g/L bacto yeast extract, 4 mUL glycerol, 2.31 g/L KH₂PO₄, 12.54 g/L K₂HPO₄) supplemented with ampicillin (100 µg/mL) was inoculated with 3 mL of TOP10 F' PQE30DeraC overnight culture. Cells were grown (37 °C, 250 rpm) until OD₆₀₀ reached approx. 0.8. Protein expression was induced with IPTG (1 mM final concentration) and cells were left in the same growing conditions for additional 4 h. The cell pelet was harvested by centrifugation (10 min, 6000 g, 4 °C).The pellet was resuspended in phosphate buffer (9mL, 50 mM NaH₂PO₄, pH 8.0, 300 mM NaCl ) giving whole cell catalyst. Alternatively the pellet was resuspended in lytic buffer (9 mL, 50 mM NaH₂PO₄, pH 8.0, 300 mM NaCl, 2 mM DTT). Cells were sonified (3 x 15 s) using Branson digital sonifier and cell debris was removed by sedimentation (10 min, 20 000 *g*, 4 °C). Clear aqueous solution of DERA 01 was thus obtained.

### Example 2

### Preparation of (4R,6S)-6-(dimethoxymethyl)tetrahydro-2H-pyran-2,4-diol [1]

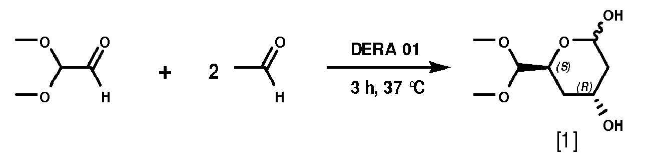

To a solution of phosphate buffer (700 mL, 50 mM NaH₂PO₄, pH 8.0, 300mM NaCl) and 2,2-dimethoxyethanal (100 mL, 0.5 M in phosphate buffer as described above) an aqueous solution of DERA 01 (100ml, prepared according to example 1) and solution of acetaldehyde (Fluka, 100 mL, 1.0 M in phosphate buffer as described above) was added. The mixture was stirred for 3 h at 37 °C and the pH was regulated at 8.0 with NaOH. The conversion of the reaction was monitored by gas chromatography (GC). After 3 h the proteins in the reaction mixture (1 L) were precipitated with acetonitrile (4 L) and the solution was filtrated using celite and glass filter. The acetonitrile was evaporated and residual water was removed with liophilization to give the crude lactol [1] (18 g), which was directly solubilized, filtered and submitted to silica-gel column (methanol/*t*-butyl methyl ether 1:1). Fractions were collected and analyzed with thin-layer silica-gel chromatography (diisopropyl ether/acetonitrile 2:1). The solvent from the fraction with the product [1] was evaporated and the dried product (154 mg, 1.6 % yield) was analyzed with ¹H and ¹³C NMR. ¹H NMR (300 MHz, CDCl₃): δ = 1.4-1.9 (m), 3.42 (s), 4.15-4.35 (m), 5.25 (d). ¹³C NMR (300 MHz, CDCl₃): δ = 32.8, 35.0, 54.5, 54.9, 62.8, 64.5, 92.9, 105.7.

### Example 3

### Preparation of (4R,6S)-6-(dimethoxymethyl)tetrahydro-2H-pyran-2,4-diol [1]

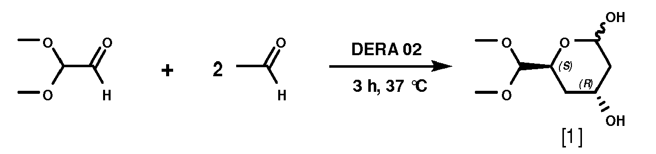

To a solution of phosphate buffer (700 mL, 50 mM NaH₂PO₄, pH 8.0, 300mM NaCl) and 2,2-dimethoxyethanal (100 mL, 0.5 M in phosphate buffer as described above) was added an aqueous solution of DERA 02 (100ml, prepared with analogue method to one described in Example 1) and solution of acetaldehyde (Fluka, 100 mL, 1.0 M in phosphate buffer as described above). The mixture was stirred for 3 h at 37 °C and the pH was kept at 8.0. The conversion of the reaction was monitored by gas chromatography (GC). After 3 h the proteins in the reaction mixture (1 L) were precipitated with acetonitrile (4 L) and the solution was filtrated using celite and glass filter. The acetonitrile was evaporated and residual water was removed with liophilization to give the crude lactol [1] (22 g), which was directly solubilized, filtered and submitted to silica-gel column (methanol/*t*-butyl methyl ether 1:1). Fractions were collected and analyzed with thin-layer silica-gel chromatography (diisopropyl ether/acetonitrile 2:1). The solvent from the fraction with the product [1] was evaporated and the dried product (2.8 g, 29 % yield) was analyzed with ¹H and ¹³C NMR. ¹H NMR (300 MHz, CDCl₃): δ = 1.4-1.9 (m), 3.42 (s), 4.15-4.35 (m), 5.25 (d). ¹³C NMR (300 MHz, CDCl₃): δ = 32.8, 35.0, 54.5, 54.9, 62.8, 64.5, 92.9, 105.7.

### Example 4

### Preparation of (4R,6S)-6-(dimethoxymethyl)tetrahydro-2H-pyran-2,4-diol [1]

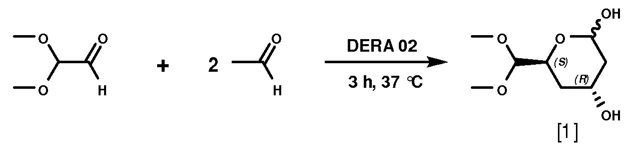

To a solution of phosphate buffer (700 mL, 50 mM NaH₂PO₄, pH 8.0, 300mM NaCl) and 2,2-dimethoxyethanal (100 mL, 1.0M in phosphate buffer as described above ) was added a whole cell catalyst with DERA 02 (100ml, prepared with analogue method to one described in Example 1 ) and solution of acetaldehyde (Fluka, 100 mL, 2.0 M in phosphate buffer as described above). The mixture was stirred for 3 h at 37 °C and the pH was kept at 8.0. The conversion of the reaction was monitored by gas chromatography (GC). After 3 h the cells were removed with sedimentation (10 min, 6000 g, 4 °C). Residual water was removed with liophilization to give the crude lactol [1] (23 g) as an oil, which was directly solubilized, filtered and submitted to silica-gel column (methanol/*t*-butyl methyl ether 1:1). Fractions were collected and analyzed with thin-layer silica-gel chromatography (diisopropyl ether/acetonitrile 2:1). The solvent from the fraction with the product [1] was evaporated and the dried product (1.8 g, 9.4 % yield) was analyzed with ¹H and ¹³C NMR. ¹H NMR (300 MHz, CDCl₃): δ = 1.4-1.9 (m), 3.42 (s), 4.15-4.35 (m), 5.25 (d). ¹³C NMR (300 MHz, CDCl₃): δ = 32.8, 35.0, 54.5, 54.9, 62.8, 64.5, 92.9, 105.7.

### Example 5

### Preparation of (4R,6S)-6-(dimethoxymethyl)tetrahydro-2H-pyran-2,4-diol [1]

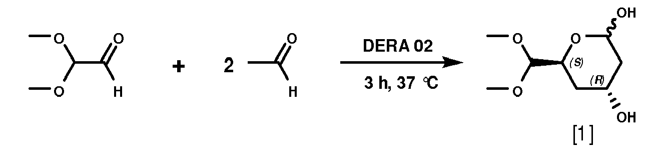

To a solution of phosphate buffer (700 mL, 50 mM NaH₂PO₄, pH 8.0) was added an aqueous solution of DERA 02 (100 mL, prepared with analogue method to one described in Example 1). A solution of 2,2-dimethoxyethanal (Aldrich, 100 mL, 1.0 M in phosphate buffer as described above) and acetaldehyde (Fluka, 100 mL, 2.0 M in phosphate buffer as described above) was continuously added separately to the above DERA solution by a programmed pump within 2 h. The mixture was stirred for 3 h at 37 °C and the pH was kept at 8.0. The conversion of the reaction was monitored by gas chromatography (GC). After 3 h the proteins in the reaction mixture (1 L) were precipitated with acetonitrile (4 L) and the solution was filtrated using celite and glass filter. The acetonitrile was evaporated and residual water was removed with liophilization to give the crude lactol [1] (15 g), which was directly solubilized, filtered and submitted to silica-gel column (methanol/*t*-butyl methyl ether 1:1). Fractions were collected and analyzed with thin-layer silica-gel chromatography (diisopropyl ether/acetonitrile 2:1). The solvent from the fraction with the product [1] was evaporated and the dried product (6.2 g, 23.3% yield) was analyzed with ¹H and ¹³C NMR. ¹H NMR (300 MHz, CDCl₃): δ = 1.4-1.9 (m), 3.42 (s), 4.15-4.35 (m), 5.25 (d). ¹³C NMR (300 MHz, CDCl₃): δ = 32.8, 35.0, 54.5, 54.9, 62.8, 64.5, 92.9, 105.7.

### Example 5

### Preparation of (4R,6S)-6-(dimethoxymethyl)tetrahydro-2H-pyran-2,4-diol [1]

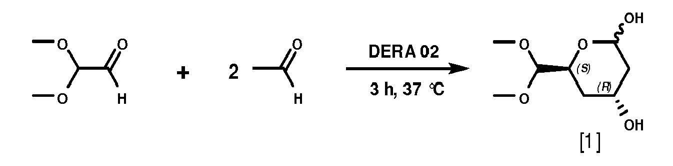

To a solution of phosphate buffer (700 mL, 50 mM NaH₂PO₄, pH 8.0) was added an aqueous solution of DERA 02 (100 mL, prepared with analogue method to one described in Example 1) and a solution of 2,2-dimethoxyethanal (Aldrich, 100 mL, 4.0 M in phosphate buffer as described above). A solution of acetaldehyde (Fluka, 100 mL, 8.0 M in phosphate buffer as described above) was continuously added to the above DERA solution by a programmed pump within 2 h. The mixture was stirred for 3 h at 37 °C and the pH was kept at 8.0. The conversion of the reaction was monitored by gas chromatography (GC). After 3 h the proteins in the reaction mixture (1 L) were precipitated with acetonitrile (4 L) and the solution was filtrated using celite and glass filter. The acetonitrile was evaporated and residual water was removed with liophilization to give the crude lactol [1] (37 g), which was directly solubilized, filtered and submitted to silica-gel column (methanol/*t*-butyl methyl ether 1:1). Fractions were collected and analyzed with thin-layer silica-gel chromatography (diisopropyl ether/acetonitrile 2:1). The solvent from the fraction with the product [1] was evaporated and the dried product (9.4 g, 12.2 % yield) was analyzed with ¹H and ¹³C NMR. ¹H NMR (300 MHz, CDCl₃): δ = 1.4-1.9 (m), 3.42 (s), 4.15-4.35 (m), 5.25 (d). ¹³C NMR (300 MHz, CDCl₃): δ = 32.8, 35.0, 54.5, 54.9, 62.8, 64.5, 92.9, 105.7.

### Example 6

### Preparation of Rosuvastatin

To a solution of 1.4 g of (4R,6S)-6-(dimethoxymethyl)-tetrahydro-2H-pyran-2,4-diol in 100 mL of water is added 2.4 g barium carbonate and 0.5 mL of bromine at 0 °C. After 3 hours of stirring at 0 - 5 °C, the product is extracted three times with 300 mL of ethyl acetate. Finally the ethyl acetate is distilled off. To the 0.5 g of thus prepared (4R,6S)-6-(dimethoxymethyl)-4-hydroxy-tetrahydropyran-2-one in 50 mL of dichloromethane, a 280 mg of imidazole is added at 25 °C and after cooling to 0 °C 400 mg of tertbutyldimethylsilyl chloride is added. The reaction is then stirred for 18 hours and dichloromethane is distilled off. To a solution of 0.40 g thus obtained (4R,6S)-4-(tert-butyldimethylsilyloxy)-6-(dimethoxymethyl)-tetrahydropyran-2-one in 12 mL of acetone a 35 mg of iodine is added at 25 °C and stirred for 48 hours. 200 mg of the obtained (4R,6S)-4-(tert-butyldimethylsilyloxy)-6-(dihydroxymethyl)tetrahydro-2H-pyran-2-one is the next step dissolved in toluene and added to a stirred suspension of ((4-(4-fluorophenyl)-6-isopropyl-2-(N-methylmethyl-sulfonamido)pyrimidin-5-yl)methyl)tributylphosphonium 2,2,2-trifluoro-acetate (504 mg) and sodium hexamethyldisilazane in dry toluene. The protecting group is removed by tetra-n-butylammonium fluoride trihydrate and the obtained compound converted to calcium salt by calcium acetate.

### Example 7

### Preparation of (S,Z)-2-(3-((1,3-dihydroxy-2-(hydroxymethyl)propan-2-yl)(3-hydroxy-4,4-dimethoxybut-1-enyl)amino)propylamino)-2-(hydroxymethyl)propane-1,3-diol [2]

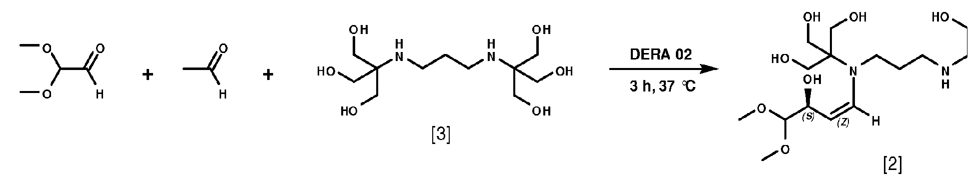

To a solution of bis-tris propane [3] buffer (70 mL, 50 mM bis-tris propane, pH 8.0, 300 mM NaCl) and a solution of 2,2-dimethoxyethanal (10 mL, 0.5 M in bis-tris propane buffer as described above) was added an aqueous solution of DERA 02 (10 mL, prepared with analogue method to one described in Example 1) and a solution of acetaldehyde (10 mL, 1.0 M in bis-tris propane buffer as described above). The mixture was stirred for 3 h at 37 °C and the pH was regulated at 8.0 with NaOH. After 3 h the proteins in the sample (1 mL) from the reaction mixture (100 mL) were precipitated with acetonitrile (9 mL) and the solution was centrifuged. Sample from supernatant was collected and analyzed directly with HPLC-MS/MS instrument. HPLC-MS/MS m/z[M+H]⁺ 413.

## Claims

1. A process comprising the step of reacting a substrate (I) with an acetaldehyde under aldolase-catalysed aldol condensation conditions to form the corresponding lactol (III) 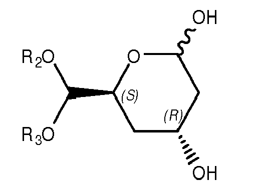 wherein the substrate (I) has formula 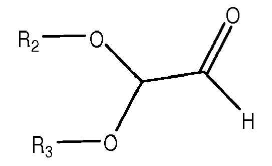 where R₂ and R₃ are independently selected from C1 - C4 alkyl, or together form a cyclic structure of formula (CH₂)ₙ where n is from 2 to 6.

2. A process for manufacturing rosuvastatin or a pharmaceuticaly acceptable salt thereof comprising the steps of
a) reacting a substrate (I) with an acetaldehyde under aldolase-catalysed aldol condensation conditions to form the corresponding lactol (III) wherein the substrate (I) has formula 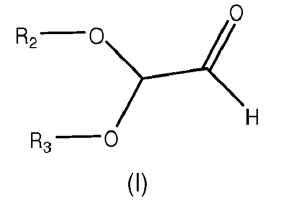 where R₂ and R₃ are independently selected from C1 - C4 alkyl, or together form a cyclic structure of formula (CH₂)ₙ where n is from 2 to 6;
b) converting said lactol into compound of formula 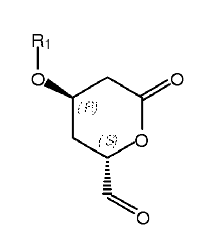
c) subjecting said compound obtained in step b) to conditions sufficient to produce rosuvastatin or a pharmaceuticaly acceptable salt thereof.

3. The process of any of claims 1 or 2, where acceptor substrate is selected among: 2,2-dimethoxyethanal (preferred), 2,2-diethoxyethanal, 2,2-dipropoxyethanal, 2,2-dibutoxyethanal, 1,3-dioxolane-2-carbaldehyde, 4-methyl-1,3-dioxolane-2-carbaldehyde, 4-ethyl-1,3-dioxolane-2-carbaldehyde" 4-propyl-1,3-dioxolane-2-carbaldehyde, 4-butyl-1,3-dioxolane-2-carbaldehyde, 4,5-dimethyl-1,3-dioxolane-2-carbaldehyde.

4. The process of claim 3 wherein said aldolase is
DERA 01 comprising a nucleotide sequence of SEQ ID NO: 1 or an amino acid sequence of SEQ ID NO: 2;
DERA 02 comprising a nucleotide sequence of SEQ ID NO: 3 or SEQ ID NO: 4 or an amino acid sequence of SEQ ID NO: 5;
DERA 03 comprising a nucleotide sequence of SEQ ID NO: 6 or an amino acid sequence of SEQ ID NO: 7;
DERA 04 comprising a nucleotide sequence of SEQ ID NO: 8 or an amino acid sequence of SEQ ID NO: 9;
DERA 05 comprising a nucleotide sequence of SEQ ID NO: 10 or an amino acid sequence of SEQ ID NO: 11;
DERA 06 comprising a nucleotide sequence of SEQ ID NO: 12 or an amino acid sequence of SEQ ID NO: 13;
DERA 07 comprising a nucleotide sequence of SEQ ID NO: 14 or an amino acid sequence of SEQ ID NO: 15;
DERA 08 comprising a nucleotide sequence of SEQ ID NO: 16 or an amino acid sequence of SEQ ID NO: 17;
DERA 09 comprising a nucleotide sequence of SEQ ID NO: 18 or an amino acid sequence of SEQ ID NO: 19;
DERA 10 comprising a nucleotide sequence of SEQ ID NO: 20 or an amino acid sequence of SEQ ID NO: 21;
DERA 11 comprising a nucleotide sequence of SEQ ID NO: 22 or an amino acid sequence of SEQ ID NO: 23;
DERA 12 comprising a nucleotide sequence of SEQ ID NO: 24 or an amino acid sequence of SEQ ID NO: 25;
DERA 13 comprising a nucleotide sequence of SEQ ID NO: 26 or an amino acid sequence of SEQ ID NO: 27;
DERA 14 comprising a nucleotide sequence of SEQ ID NO: 28 or an amino acid sequence of SEQ ID NO: 29;
DERA 15 comprising a nucleotide sequence of SEQ ID NO: 30 or an amino acid sequence of SEQ ID NO: 31;
DERA 16 comprising a nucleotide sequence of SEQ ID NO: 32 or an amino acid sequence of SEQ ID NO: 33;
DERA 17 comprising a nucleotide sequence of SEQ ID NO: 34 or an amino acid sequence of SEQ ID NO: 35;
DERA 18 is an aldolase comprising a nucleotide sequence of SEQ ID NO: 36 or an amino acid sequence of SEQ ID NO: 37;
DERA 19 comprising a nucleotide sequence of SEQ ID NO: 38 or an amino acid sequence of SEQ ID NO: 39;
DERA 20 comprising a nucleotide sequence of SEQ ID NO: 40 or an amino acid sequence of SEQ ID NO: 41;
DERA 21 comprising a nucleotide sequence of SEQ ID NO: 42 or an amino acid sequence of SEQ ID NO: 43;
DERA 22 comprising a nucleotide sequence of SEQ ID NO: 44 or an amino acid sequence of SEQ ID NO: 45;
DERA 23 comprising a nucleotide sequence of SEQ ID NO: 46 or an amino acid sequence of SEQ ID NO: 47; or an aldolase having an amino acid sequence identity of at least about 70% to amino acid sequence of any of said aldolases

5. The process of previous claim wherein said aldolase has an amino acid sequence identity of at least about 70% to amino acid sequence of SEQ ID NO: 2.

6. The process of claim 4 wherein said aldolase has an amino acid sequence identity of at least 90% to amino acid sequence of SEQ ID NO: 5.

7. The process of claim 4 wherein said aldolase has an amino acid sequence identity of at least 90% to amino acid sequence of SEQ ID NO: 17.

8. The process according to any of the previous claims wherein pH for aldolase-catalysed aldol condensation is maintained in range 6 to 10.

9. The process of previous claim wherein pH is maintained with a buffer in pH range 7 to 9.

10. Use of phosphate buffer for the process according to any of the previous claims.

11. Use of aldolase for the reaction of a substrate (I) with an acetaldehyde under aldolase-catalysed aldol condensation conditions to form the corresponding lactol (III) wherein the substrate (I) has formula 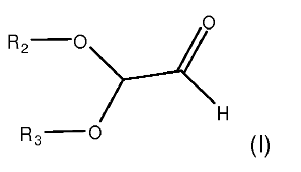 where R₂ and R₃ are independently selected from C1 - C4 alkyl, or together form a cyclic structure of formula (CH₂)ₙ where n is from 2 to 6.

12. The use according to previous claim, wherein said aldolase is 2-deoxyribose-5-phosphate aldolase.

13. The use according to previous claim, wherein aldolase is
DERA 01 comprising a nucleotide sequence of SEQ ID NO: 1 or an amino acid sequence of SEQ ID NO: 2;
DERA 02 comprising a nucleotide sequence of SEQ ID NO: 3 or SEQ ID NO: 4 or an amino acid sequence of SEQ ID NO: 5;
DERA 03 comprising a nucleotide sequence of SEQ ID NO: 6 or an amino acid sequence of SEQ ID NO: 7;
DERA 04 comprising a nucleotide sequence of SEQ ID NO: 8 or an amino acid sequence of SEQ ID NO: 9;
DERA 05 comprising a nucleotide sequence of SEQ ID NO: 10 or an amino acid sequence of SEQ ID NO: 11;
DERA 06 comprising a nucleotide sequence of SEQ ID NO: 12 or an amino acid sequence of SEQ ID NO: 13;
DERA 07 comprising a nucleotide sequence of SEQ ID NO: 14 or an amino acid sequence of SEQ ID NO: 15;
DERA 08 comprising a nucleotide sequence of SEQ ID NO: 16 or an amino acid sequence of SEQ ID NO: 17;
DERA 09 comprising a nucleotide sequence of SEQ ID NO: 18 or an amino acid sequence of SEQ ID NO: 19;
DERA 10 comprising a nucleotide sequence of SEQ ID NO: 20 or an amino acid sequence of SEQ ID NO: 21;
DERA 11 comprising a nucleotide sequence of SEQ ID NO: 22 or an amino acid sequence of SEQ ID NO: 23;
DERA 12 comprising a nucleotide sequence of SEQ ID NO: 24 or an amino acid sequence of SEQ ID NO: 25;
DERA 13 comprising a nucleotide sequence of SEQ ID NO: 26 or an amino acid sequence of SEQ ID NO: 27;
DERA 14 comprising a nucleotide sequence of SEQ ID NO: 28 or an amino acid sequence of SEQ ID NO: 29;
DERA 15 comprising a nucleotide sequence of SEQ ID NO: 30 or an amino acid sequence of SEQ ID NO: 31;
DERA 16 comprising a nucleotide sequence of SEQ ID NO: 32 or an amino acid sequence of SEQ ID NO: 33;
DERA 17 comprising a nucleotide sequence of SEQ ID NO: 34 or an amino acid sequence of SEQ ID NO: 35;
DERA 18 is an aldolase comprising a nucleotide sequence of SEQ ID NO: 36 or an amino acid sequence of SEQ ID NO: 37;
DERA 19 comprising a nucleotide sequence of SEQ ID NO: 38 or an amino acid sequence of SEQ ID NO: 39;
DERA 20 comprising a nucleotide sequence of SEQ ID NO: 40 or an amino acid sequence of SEQ ID NO: 41;
DERA 21 comprising a nucleotide sequence of SEQ ID NO: 42 or an amino acid sequence of SEQ ID NO: 43;
DERA 22 comprising a nucleotide sequence of SEQ ID NO: 44 or an amino acid sequence of SEQ ID NO: 45;
DERA 23 comprising a nucleotide sequence of SEQ ID NO: 46 or an amino acid sequence of SEQ ID NO: 47.

14. The use according to any of previous 2 claims, wherein said aldolase is comprised within living whole cell, or is comprised within inactivated whole cell, or is comprised within homogenized whole cell, or is comprised within cell free extract, or is purified enzyme, or is immobilized, or is in form of extracelularly expressed protein.

15. A process for preparing the compound of formula (III) 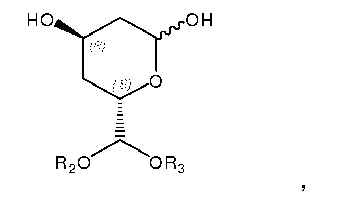 wherein R₂ and R₃ are independently selected from C1 - C4 alkyl, or together form a cyclic structure of formula (CH₂)ₙ where n is from 2 to 6,
consisting of steps of:
a) admixing the substrate of formula (I) 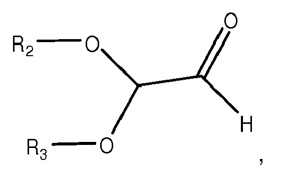 wherein R₂ and R₃ are independently selected from C1 - C4 alkyl, or together form a cyclic structure of formula (CH₂)ₙ where n is from 2 to 6,
with
acetaldehyde and
2-deoxyribose-5-phosphate aldolase
in an aqueous medium at pH value of 7 to 9 at temperature at abput 30 - 50 ° C to form a reaction mixture;
b) maintaining said reaction mixture at said temperature and pH value for time period between 1 h and 6 h; and
c) recovering said compound of formula (III),

16. A compound of formula: 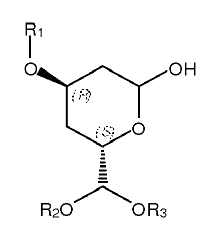 where R₁ is H or a protecting group; and R₂ and R₃ are independently selected from C1 - C4 alkyl, or together form a cyclic structure of formula (CH₂)ₙ where n is from 2 to 6.

## Amended claims

### Amended claims in accordance with Rule 137(2) EPC.

**1.** A process comprising the step of reacting a substrate (I) with an acetaldehyde under aldolase-catalysed aldol condensation conditions to form the corresponding lactol (III) 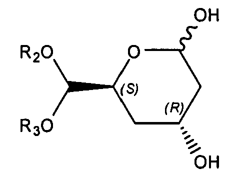 wherein the substrate (I) has formula 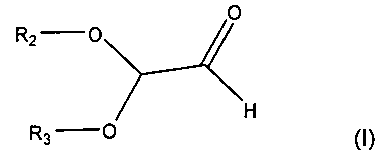 where R₂ and R₃ are independently selected from C1 - C4 alkyl, or together form a cyclic structure of formula (CH₂)ₙ where n is from 2 to 6.

**2.** A process for manufacturing rosuvastatin or a pharmaceuticaly acceptable salt thereof comprising the steps of
a) reacting a substrate (I) with an acetaldehyde under aldolase-catalysed aldol condensation conditions to form the corresponding lactol (III) wherein the substrate (I) has formula 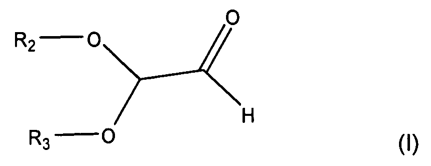 where R₂ and R₃ are independently selected from C1 - C4 alkyl, or together form a cyclic structure of formula (CH₂)ₙ where n is from 2 to 6;
b) converting said lactol into compound of formula 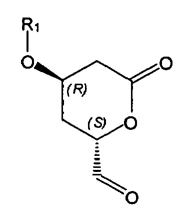
c) subjecting said compound obtained in step b) to conditions sufficient to produce rosuvastatin or a pharmaceuticaly acceptable salt thereof.

**3.** The process of any of claims 1 or 2, where acceptor substrate is selected among: 2,2-dimethoxyethanal (preferred), 2,2-diethoxyethanal, 2,2-dipropoxyethanal, 2,2-dibutoxyethanal, 1,3-dioxolane-2-carbaldehyde, 4-methyl-1,3-dioxolane-2-carbaldehyde, 4-ethyl-1,3-dioxolane-2-carbaldehyde" 4-propyl-1,3-dioxolane-2-carbaldehyde, 4-butyl-1,3-dioxolane-2-carbaldehyde, 4,5-dimethyl-1,3-dioxolane-2-carbaldehyde.

**4.** The process of claim 3 wherein said aldolase is
DERA 01 comprising a nucleotide sequence of SEQ ID NO: 1 or an amino acid sequence of SEQ ID NO: 2;
DERA 02 comprising a nucleotide sequence of SEQ ID NO: 3 or SEQ ID NO: 4 or an amino acid sequence of SEQ ID NO: 5;
DERA 03 comprising a nucleotide sequence of SEQ ID NO: 6 or an amino acid sequence of SEQ ID NO: 7;
DERA 04 comprising a nucleotide sequence of SEQ ID NO: 8 or an amino acid sequence of SEQ ID NO: 9;
DERA 05 comprising a nucleotide sequence of SEQ ID NO: 10 or an amino acid sequence of SEQ ID NO: 11;
DERA 06 comprising a nucleotide sequence of SEQ ID NO: 12 or an amino acid sequence of SEQ ID NO: 13;
DERA 07 comprising a nucleotide sequence of SEQ ID NO: 14 or an amino acid sequence of SEQ ID NO: 15;
DERA 08 comprising a nucleotide sequence of SEQ ID NO: 16 or an amino acid sequence of SEQ ID NO: 17;
DERA 09 comprising a nucleotide sequence of SEQ ID NO: 18 or an amino acid sequence of SEQ ID NO: 19;
DERA 10 comprising a nucleotide sequence of SEQ ID NO: 20 or an amino acid sequence of SEQ ID NO: 21;
DERA 11 comprising a nucleotide sequence of SEQ ID NO: 22 or an amino acid sequence of SEQ ID NO: 23;
DERA 12 comprising a nucleotide sequence of SEQ ID NO: 24 or an amino acid sequence of SEQ ID NO: 25;
DERA 13 comprising a nucleotide sequence of SEQ ID NO: 26 or an amino acid sequence of SEQ ID NO: 27;
DERA 14 comprising a nucleotide sequence of SEQ ID NO: 28 or an amino acid sequence of SEQ ID NO: 29;
DERA 15 comprising a nucleotide sequence of SEQ ID NO: 30 or an amino acid sequence of SEQ ID NO: 31;
DERA 16 comprising a nucleotide sequence of SEQ ID NO: 32 or an amino acid sequence of SEQ ID NO: 33;
DERA 17 comprising a nucleotide sequence of SEQ ID NO: 34 or an amino acid sequence of SEQ ID NO: 35;
DERA 18 is an aldolase comprising a nucleotide sequence of SEQ ID NO: 36 or an amino acid sequence of SEQ ID NO: 37;
DERA 19 comprising a nucleotide sequence of SEQ ID NO: 38 or an amino acid sequence of SEQ ID NO: 39;
DERA 20 comprising a nucleotide sequence of SEQ ID NO: 40 or an amino acid sequence of SEQ ID NO: 41;
DERA 21 comprising a nucleotide sequence of SEQ ID NO: 42 or an amino acid sequence of SEQ ID NO: 43;
DERA 22 comprising a nucleotide sequence of SEQ ID NO: 44 or an amino acid sequence of SEQ ID NO: 45;
DERA 23 comprising a nucleotide sequence of SEQ ID NO: 46 or an amino acid sequence of SEQ ID NO: 47; or an aldolase having an amino acid sequence identity of at least about 70% to amino acid sequence of any of said aldolases

**5.** The process of previous claim wherein said aldolase has an amino acid sequence identity of at least about 70% to amino acid sequence of SEQ ID NO: 2.

**6.** The process of claim 4 wherein said aldolase has an amino acid sequence identity of at least 90% to amino acid sequence of SEQ ID NO: 5.

**7.** The process of claim 4 wherein said aldolase has an amino acid sequence identity of at least 90% to amino acid sequence of SEQ ID NO: 17.

**8.** The process according to any of the previous claims wherein pH for aldolase-catalysed aldol condensation is maintained in range 6 to 10.

**9.** The process of previous claim wherein pH is maintained with a buffer in pH range 7 to 9.

**10.** The process according to any of previous claims, wherein said aldolase is comprised within living whole cell, or is comprised within inactivated whole cell, or is comprised within homogenized whole cell, or is comprised within cell free extract, or is purified enzyme, or is immobilized, or is in form of extracelularly expressed protein.

**11.** Use of phosphate buffer for the process according to any of the previous claims.

**12.** A process according to claim 1 for preparing the compound of formula (III) 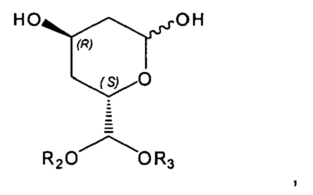 wherein R₂ and R₃ are independently selected from C1 - C4 alkyl, or together form a cyclic structure of formula (CH₂)ₙ where n is from 2 to 6, consisting of steps of:
a) admixing the substrate of formula (I) 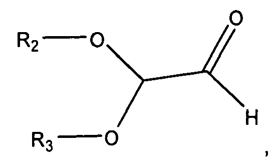 wherein R₂ and R₃ are independently selected from C1 - C4 alkyl, or together form a cyclic structure of formula (CH₂)ₙ where n is from 2 to 6,
with
acetaldehyde and
2-deoxyribose-5-phosphate aldolase
in an aqueous medium at pH value of 7 to 9 at temperature at 30 - 50° C to form a reaction mixture;
b) maintaining said reaction mixture at said temperature and pH value for time period between 1 h and 6 h; and
c) recovering said compound of formula (III).


SEQUENCE LISTING

<110>  Lek Pharmaceuticals
       Mrak, Peter

<120>  SYNTHESIS OF STATINS

<130>  50851

<160>  49

<170>  PatentIn version 3.4

<210>  1
<211>  780
<212>  DNA
<213>  Escherichia coli

<400>  1
atgactgatc tgaaagcaag cagcctgcgt gcactgaaat tgatggacct gaccaccctg      60

aatgacgacg acaccgacga gaaagtgatc gccctgtgtc atcaggccaa aactccggtc     120

ggcaataccg ccgctatctg tatctatcct cgctttatcc cgattgctcg caaaactctg     180

aaagagcagg gcaccccgga aatccgtatc gctacggtaa ccaacttccc acacggtaac     240

gacgacatcg acatcgcgct ggcagaaacc cgtgcggcaa tcgcctacgg tgctgatgaa     300

gttgacgttg tgttcccgta ccgcgcgctg atggcgggta acgagcaggt tggttttgac     360

ctggtgaaag cctgtaaaga ggcttgcgcg gcagcgaatg tactgctgaa agtgatcatc     420

gaaaccggcg aactgaaaga cgaagcgctg atccgtaaag cgtctgaaat ctccatcaaa     480

gcgggtgcgg acttcatcaa aacctctacc ggtaaagtgg ctgtgaacgc gacgccggaa     540

agcgcgcgca tcatgatgga agtgatccgt gatatgggcg tagaaaaaac cgttggtttc     600

aaaccggcgg gcggcgtgcg tactgcggaa gatgcgcaga aatatctcgc cattgcagat     660

gaactgttcg gtgctgactg ggcagatgcg cgtcactacc gctttggcgc ttccagcctg     720

ctggcaagcc tgctgaaagc gctgggtcac ggcgacggta agagcgccag cagctactaa     780

<210>  2
<211>  259
<212>  PRT
<213>  Escherichia coli

<400>  2

Met Thr Asp Leu Lys Ala Ser Ser Leu Arg Ala Leu Lys Leu Met Asp
1               5                   10                  15

Leu Thr Thr Leu Asn Asp Asp Asp Thr Asp Glu Lys Val Ile Ala Leu
            20                  25                  30

Cys His Gln Ala Lys Thr Pro Val Gly Asn Thr Ala Ala Ile Cys Ile
        35                  40                  45

Tyr Pro Arg Phe Ile Pro Ile Ala Arg Lys Thr Leu Lys Glu Gln Gly
    50                  55                  60

Thr Pro Glu Ile Arg Ile Ala Thr Val Thr Asn Phe Pro His Gly Asn
65                  70                  75                  80

Asp Asp Ile Asp Ile Ala Leu Ala Glu Thr Arg Ala Ala Ile Ala Tyr
                85                  90                  95

Gly Ala Asp Glu Val Asp Val Val Phe Pro Tyr Arg Ala Leu Met Ala
            100                 105                 110

Gly Asn Glu Gln Val Gly Phe Asp Leu Val Lys Ala Cys Lys Glu Ala
        115                 120                 125

Cys Ala Ala Ala Asn Val Leu Leu Lys Val Ile Ile Glu Thr Gly Glu
    130                 135                 140

Leu Lys Asp Glu Ala Leu Ile Arg Lys Ala Ser Glu Ile Ser Ile Lys
145                 150                 155                 160

Ala Gly Ala Asp Phe Ile Lys Thr Ser Thr Gly Lys Val Ala Val Asn
                165                 170                 175

Ala Thr Pro Glu Ser Ala Arg Ile Met Met Glu Val Ile Arg Asp Met
            180                 185                 190

Gly Val Glu Lys Thr Val Gly Phe Lys Pro Ala Gly Gly Val Arg Thr
        195                 200                 205

Ala Glu Asp Ala Gln Lys Tyr Leu Ala Ile Ala Asp Glu Leu Phe Gly
    210                 215                 220

Ala Asp Trp Ala Asp Ala Arg His Tyr Arg Phe Gly Ala Ser Ser Leu
225                 230                 235                 240

Leu Ala Ser Leu Leu Lys Ala Leu Gly His Gly Asp Gly Lys Ser Ala
                245                 250                 255

Ser Ser Tyr


<210>  3
<211>  663
<212>  DNA
<213>  Unknown

<220>
<223>  "Cloned"

<400>  3
atgaatatcg cgaaaatgat cgatcatacg ctgctcaaac cggaagcgac agaacaacaa      60

atcgtgcaac tgtgcacgga agcaaagcaa tacggctttg ctgccgtgtg cgtcaaccca     120

acgtgggtga aaacggcggc gcgcgagctt tccggcacgg atgtccgcgt ctgcacggtc     180

atcggctttc cacttggggc aacgacgccg gaaacaaagg cgtttgaaac aacgaacgcc 240

atcgaaaacg gcgctcgcga agtcgacatg gtgatcaaca tcggcgcgtt aaaaagcggg 300

caagacgagc ttgtcgagcg cgacattcgt gcggttgtcg aagcggcggc tggcagggcg 360

cttgtcaaag tgatcgttga aacggcgctt ttgaccgatg aggaaaaagt gcgcgcctgc 420

cagctcgcag tgaaagccgg cgctgattat gtgaaaacgt cgaccgggtt ttccggcgga 480

ggtgcgacgg tggaggatgt ggcgctgatg cggaaaacgg tcggcgacag agcaggcgtc 540

aaagcatcag gcggcgtccg tgactggaaa accgctgagg cgatgatcaa cgccggcgcg 600

acgcgcatcg gcacaagctc tggggtggcg atcgtcaccg gcgggacggg ccgcgctgac 660

tac 663

<210> 4
<211> 663
<212> DNA
<213> Unknown

<220>
<223> "Cloned"

<400> 4
atgaacatcg cgaaaatgat cgatcacacc ctgctgaaac cggaagcgac cgaacagcag 60

attgttcagc tgtgcaccga agcgaaacag tatggttttg cggcggtgtg tgttaatccg 120

acctgggtta aaaccgcggc gcgtgaactg agcggcaccg atgttcgtgt gtgcaccgtg 180

attggttttc cgctgggtgc gaccaccccg gaaaccaaag cgtttgaaac caccaacgcg 240

attgaaaatg gtgcgcgcga agtggatatg gtgattaaca tcggcgcgct gaaaagcggt 300

caggatgaac tggttgaacg cgatattcgt gcggttgttg aagcggcggc gggtcgcgcg 360

ctggttaaag tgattgtgga aaccgcgctg ctgaccgatg aagaaaaagt gcgtgcctgt 420

cagctggcgg ttaaagcggg tgcggattac gttaaaacca gcaccggttt tagcggtggt 480

ggtgcgaccg ttgaagatgt tgcgctgatg cgtaaaaccg ttggtgatcg tgcgggtgtg 540

aaagcgagcg gtggtgttcg cgattggaaa accgcggaag cgatgattaa tgcgggcgcg 600

acccgtattg gcaccagcag cggtgttgcg attgttaccg gtggcaccgg tcgtgcggat 660

tat 663

<210> 5
<211> 221
<212> PRT
<213> Unknown

<220>
<223> testek

<400> 5

Met Asn Ile Ala Lys Met Ile Asp His Thr Leu Leu Lys Pro Glu Ala
1               5                   10                  15

Thr Glu Gln Gln Ile Val Gln Leu Cys Thr Glu Ala Lys Gln Tyr Gly

20                  25                  30

Phe Ala Ala Val Cys Val Asn Pro Thr Trp Val Lys Thr Ala Ala Arg
        35                  40                  45

Glu Leu Ser Gly Thr Asp Val Arg Val Cys Thr Val Ile Gly Phe Pro
    50                  55                  60

Leu Gly Ala Thr Thr Pro Glu Thr Lys Ala Phe Glu Thr Thr Asn Ala
65                  70                  75                  80

Ile Glu Asn Gly Ala Arg Glu Val Asp Met Val Ile Asn Ile Gly Ala
                85                  90                  95

Leu Lys Ser Gly Gln Asp Glu Leu Val Glu Arg Asp Ile Arg Ala Val
            100                 105                 110

Val Glu Ala Ala Ala Gly Arg Ala Leu Val Lys Val Ile Val Glu Thr
        115                 120                 125

Ala Leu Leu Thr Asp Glu Glu Lys Val Arg Ala Cys Gln Leu Ala Val
    130                 135                 140

Lys Ala Gly Ala Asp Tyr Val Lys Thr Ser Thr Gly Phe Ser Gly Gly
145                 150                 155                 160

Gly Ala Thr Val Glu Asp Val Ala Leu Met Arg Lys Thr Val Gly Asp
                165                 170                 175

Arg Ala Gly Val Lys Ala Ser Gly Gly Val Arg Asp Trp Lys Thr Ala
            180                 185                 190

Glu Ala Met Ile Asn Ala Gly Ala Thr Arg Ile Gly Thr Ser Ser Gly
        195                 200                 205

Val Ala Ile Val Thr Gly Gly Thr Gly Arg Ala Asp Tyr
    210                 215                 220

<210>  6
<211>  666
<212>  DNA
<213>  Oceanobacillus iheyensis

<400>  6
atggatttag ctaaatatat tgatcatact caattaaaac cagatactac aaaacaaagt      60

attgtaaaaa ttgtggaaga ggcaaaacaa catgaatttg cttcagtatg tgttaatcca     120

cactgggttt cttactgtta taatgaatta aaagatacac cagttaaagt ttgtacagta     180

attggattcc cattaggagc cacttctact gaaacgaaaa tttttgaaac caatcaggct     240

attgctgatg gtgctacaga agtagacatg gtaattaatg tcggtgaatt aaaatcgaat     300

aatgatgctt ttgttgaaaa agacatccgt gctgttgttg aagcagcaaa aggtaaagct     360

ttaacaaaag tgataattga aacaagtctt ttaacagaag atgaaaaagt acgtgcttgt 420

aaattagcaa aaaatgcaga ggctgactat gtaaaaactt ctactgggtt ctctggtggc 480

ggcgcaactg ttgaggatat tcgcttaatg cgagagacag taggacctga aatgggagtg 540

aaagcatctg gtggtgttcg tgatttagag caaacagaag caatgattga agctggagca 600

actagaattg gagctagttc tggggtagcg attgtctcag gagaacaagg tacatcagat 660

tactaa 666

<210> 7
<211> 221
<212> PRT
<213> Oceanobacillus iheyensis

<400> 7

Met Asp Leu Ala Lys Tyr Ile Asp His Thr Gln Leu Lys Pro Asp Thr
1               5                   10                  15

Thr Lys Gln Ser Ile Val Lys Ile Val Glu Glu Ala Lys Gln His Glu
            20                  25                  30

Phe Ala Ser Val Cys Val Asn Pro His Trp Val Ser Tyr Cys Tyr Asn
        35                  40                  45

Glu Leu Lys Asp Thr Pro Val Lys Val Cys Thr Val Ile Gly Phe Pro
    50                  55                  60

Leu Gly Ala Thr Ser Thr Glu Thr Lys Ile Phe Glu Thr Asn Gln Ala
65                  70                  75                  80

Ile Ala Asp Gly Ala Thr Glu Val Asp Met Val Ile Asn Val Gly Glu
                85                  90                  95

Leu Lys Ser Asn Asn Asp Ala Phe Val Glu Lys Asp Ile Arg Ala Val
            100                 105                 110

Val Glu Ala Ala Lys Gly Lys Ala Leu Thr Lys Val Ile Ile Glu Thr
        115                 120                 125

Ser Leu Leu Thr Glu Asp Glu Lys Val Arg Ala Cys Lys Leu Ala Lys
    130                 135                 140

Asn Ala Glu Ala Asp Tyr Val Lys Thr Ser Thr Gly Phe Ser Gly Gly
145                 150                 155                 160

Gly Ala Thr Val Glu Asp Ile Arg Leu Met Arg Glu Thr Val Gly Pro
                165                 170                 175

Glu Met Gly Val Lys Ala Ser Gly Gly Val Arg Asp Leu Glu Gln Thr
            180                 185                 190

Glu Ala Met Ile Glu Ala Gly Ala Thr Arg Ile Gly Ala Ser Ser Gly
        195                 200                 205

Val Ala Ile Val Ser Gly Glu Gln Gly Thr Ser Asp Tyr
    210                 215                 220

<210>  8
<211>  663
<212>  DNA
<213>  Deinococcus radiodurans

<400>  8
atgtcactcg cctcctacat cgaccacacg ctgcttaagg ccaccgccac gctcgccgac      60

atccgcacgc tgtgtgagga agcccgcgag cactcgttct acgcggtgtg catcaacccg     120

gtctttattc cccacgcccg cgcctggctc gaaggcagcg acgtgaaggt cgccaccgtc     180

tgcggctttc ccctcggcgc catcagctcc gagcagaaag ctctggaagc ccgcctgagc     240

gccgaaacgg gcgccgacga aatcgatatg gtcatccaca tcggctcggc gcttgccggc     300

gactgggacg cggtggaagc cgacgtgcgg gcagtgcgcc gcgcggtgcc cgagcaggtg     360

ctcaaggtga ttatcgaaac ctgctacctg accgacgagc aaaagcgctt ggcgactgag     420

gtcgccgtac agggcggcgc cgacttcgtg aagacgagca caggcttcgg caccggcggc     480

gccaccgtgg acgacgtgcg cctgatggcg gaagtgatcg ggggccgcgc cggactcaag      540

gcggcgggcg gcgtccgcac tcctgccgac gcgcaagcca tgatcgaggc gggcgcgacc     600

cggctgggca cctcgggcgg cgtgggtctg gtgtcgggcg gcgaaaacgg agccggctac     660

tga                                                                   663

<210>  9
<211>  220
<212>  PRT
<213>  Deinococcus radiodurans

<400>  9

Met Ser Leu Ala Ser Tyr Ile Asp His Thr Leu Leu Lys Ala Thr Ala
1               5                   10                  15

Thr Leu Ala Asp Ile Arg Thr Leu Cys Glu Glu Ala Arg Glu His Ser
            20                  25                  30

Phe Tyr Ala Val Cys Ile Asn Pro Val Phe Ile Pro His Ala Arg Ala
        35                  40                  45

Trp Leu Glu Gly Ser Asp Val Lys Val Ala Thr Val Cys Gly Phe Pro
    50                  55                  60

Leu Gly Ala Ile Ser Ser Glu Gln Lys Ala Leu Glu Ala Arg Leu Ser
65                  70                  75                  80

Ala Glu Thr Gly Ala Asp Glu Ile Asp Met Val Ile His Ile Gly Ser

85                  90                  95

Ala Leu Ala Gly Asp Trp Asp Ala Val Glu Ala Asp Val Arg Ala Val
            100                 105                 110

Arg Arg Ala Val Pro Glu Gln Val Leu Lys Val Ile Ile Glu Thr Cys
        115                 120                 125

Tyr Leu Thr Asp Glu Gln Lys Arg Leu Ala Thr Glu Val Ala Val Gln
    130                 135                 140

Gly Gly Ala Asp Phe Val Lys Thr Ser Thr Gly Phe Gly Thr Gly Gly
145                 150                 155                 160

Ala Thr Val Asp Asp Val Arg Leu Met Ala Glu Val Ile Gly Gly Arg
                165                 170                 175

Ala Gly Leu Lys Ala Ala Gly Gly Val Arg Thr Pro Ala Asp Ala Gln
            180                 185                 190

Ala Met Ile Glu Ala Gly Ala Thr Arg Leu Gly Thr Ser Gly Gly Val
        195                 200                 205

Gly Leu Val Ser Gly Gly Glu Asn Gly Ala Gly Tyr
    210                 215                 220

<210>  10
<211>  675
<212>  DNA
<213>  Propionibacterium acnes

<400>  10
atgtcctctg ccccactgtc tgccaccgag ttggccggca tgatcgatca caccctgctg      60

acccctgagg ccacccacaa cgacgtcgcc aagctggtcg ccgatgccaa aaaatatggg     120

acgtggtcgg tgtgcgtatc gccatcgatg ctgccgttga acctcgacat gggtgacgtg     180

catctggccg tcgtgtgcgg gtttccgtca ggcaagcaca ccagcgcagt aaaggctgct     240

gaggctcgtg aggccatcgc cgcaggggcc gaggaggtcg acatggtgat caaccttggt     300

ctggtaaagg agggacgctg ggaggacgtc accgccgata tcgctgccgt caagcaggcc     360

gtcccggatc cgaagatcct taaggtcatt atcgagtcgg cggtgctgac cgacgacgag     420

atcgtgcggg catgccaggc tgccgagaag gccggcgccg acttcgtcaa gacgtcgacg     480

ggattccacc cacgtggcgg cgcaagcgtc gaggccgtca aggtcatggc tgacactgtt     540

ggtggacgtc tgggcgtcaa agcgtccggc ggcatccgcg actaccagac ggcatgcgcg     600

atggtcgagg ccggggcgac gcgtctagga gtttcctcga ccgccaagat ccttgccgga     660

gctcccacgg agtga                                                      675

<210>  11
<211>  224

<212>  PRT
<213>  Propionibacterium acnes

<400>  11

Met Ser Ser Ala Pro Leu Ser Ala Thr Glu Leu Ala Gly Met Ile Asp
1               5                   10                  15

His Thr Leu Leu Thr Pro Glu Ala Thr His Asn Asp Val Ala Lys Leu
            20                  25                  30

Val Ala Asp Ala Lys Lys Tyr Gly Thr Trp Ser Val Cys Val Ser Pro
        35                  40                  45

Ser Met Leu Pro Leu Asn Leu Asp Met Gly Asp Val His Leu Ala Val
    50                  55                  60

Val Cys Gly Phe Pro Ser Gly Lys His Thr Ser Ala Val Lys Ala Ala
65                  70                  75                  80

Glu Ala Arg Glu Ala Ile Ala Ala Gly Ala Glu Glu Val Asp Met Val
                85                  90                  95

Ile Asn Leu Gly Leu Val Lys Glu Gly Arg Trp Glu Asp Val Thr Ala
            100                 105                 110

Asp Ile Ala Ala Val Lys Gln Ala Val Pro Asp Pro Lys Ile Leu Lys
        115                 120                 125

Val Ile Ile Glu Ser Ala Val Leu Thr Asp Asp Glu Ile Val Arg Ala
    130                 135                 140

Cys Gln Ala Ala Glu Lys Ala Gly Ala Asp Phe Val Lys Thr Ser Thr
145                 150                 155                 160

Gly Phe His Pro Arg Gly Gly Ala Ser Val Glu Ala Val Lys Val Met
                165                 170                 175

Ala Asp Thr Val Gly Gly Arg Leu Gly Val Lys Ala Ser Gly Gly Ile
            180                 185                 190

Arg Asp Tyr Gln Thr Ala Cys Ala Met Val Glu Ala Gly Ala Thr Arg
        195                 200                 205

Leu Gly Val Ser Ser Thr Ala Lys Ile Leu Ala Gly Ala Pro Thr Glu
    210                 215                 220


<210>  12
<211>  768
<212>  DNA
<213>  Chromobacterium violaceum

<400>  12
atgtctgcac tgattgaagc cgcgcgccgc gcgctgtccc tgatggacct gaccaccctc      60

aacgacgacg ataccgacga gaaggtggcc gcgctgtgcc gcaaggccaa gagcccggac     120

ggcaccgtgg cggcggtatg cgtgtttccc cgcttcgtgc ccatcgccaa gaagacgctg     180

cgcgaagcgg gttgtccgga ggtgcaggtg gccaccgtca ccaacttccc gcacggcaat     240

gacgacgtct ccatcgcggt ggccgaaacc cgcgccgcca tcgcctacgg cgccgacgaa     300

gtggacgtgg tgttcccgta ccgcgcgctg atggccggca accgcgacat cggcttcgag     360

ctggtcaagg cctgcaagga agcctgcggc ggcaagctct tgaaagtgat catcgagagc     420

ggcgaactga aggacgcggc gctgatccgc gaagccagcg agatttccat ccgcgccggg     480

gccgacttca tcaagacttc caccggcaag gtgccggtca acgccacctt gcccgcggcc     540

gagaccatgc tggccgtgat caaggagcag ggcggccagt gcggcttcaa ggccgccggc     600

ggcgtcaaga gcgccaccga ggcggccgaa tacctggccc tggccgcgcg cctgctgggc     660

gaagattggg tgagcgcccg ccacttccgc ttcggcgcgt ccagcctgct ggccaatctg     720

cagatcgaga tcgccggcgg cgtcgccaag ccgagcagcg gctactga                 768

<210>  13
<211>  255
<212>  PRT
<213>  Chromobacterium violaceum

<400>  13

Met Ser Ala Leu Ile Glu Ala Ala Arg Arg Ala Leu Ser Leu Met Asp
1               5                   10                  15

Leu Thr Thr Leu Asn Asp Asp Asp Thr Asp Glu Lys Val Ala Ala Leu
            20                  25                  30

Cys Arg Lys Ala Lys Ser Pro Asp Gly Thr Val Ala Ala Val Cys Val
        35                  40                  45

Phe Pro Arg Phe Val Pro Ile Ala Lys Lys Thr Leu Arg Glu Ala Gly
    50                  55                  60

Cys Pro Glu Val Gln Val Ala Thr Val Thr Asn Phe Pro His Gly Asn
65                  70                  75                  80

Asp Asp Val Ser Ile Ala Val Ala Glu Thr Arg Ala Ala Ile Ala Tyr
                85                  90                  95

Gly Ala Asp Glu Val Asp Val Val Phe Pro Tyr Arg Ala Leu Met Ala
            100                 105                 110

Gly Asn Arg Asp Ile Gly Phe Glu Leu Val Lys Ala Cys Lys Glu Ala
        115                 120                 125

Cys Gly Gly Lys Leu Leu Lys Val Ile Ile Glu Ser Gly Glu Leu Lys
    130                 135                 140

Asp Ala Ala Leu Ile Arg Glu Ala Ser Glu Ile Ser Ile Arg Ala Gly
145                 150                 155                 160

Ala Asp Phe Ile Lys Thr Ser Thr Gly Lys Val Pro Val Asn Ala Thr
                165                 170                 175

Leu Pro Ala Ala Glu Thr Met Leu Ala Val Ile Lys Glu Gln Gly Gly
            180                 185                 190

Gln Cys Gly Phe Lys Ala Ala Gly Gly Val Lys Ser Ala Thr Glu Ala
        195                 200                 205

Ala Glu Tyr Leu Ala Leu Ala Ala Arg Leu Leu Gly Glu Asp Trp Val
    210                 215                 220

Ser Ala Arg His Phe Arg Phe Gly Ala Ser Ser Leu Leu Ala Asn Leu
225                 230                 235                 240

Gln Ile Glu Ile Ala Gly Gly Val Ala Lys Pro Ser Ser Gly Tyr
                245                 250                 255

<210>  14
<211>  780
<212>  DNA
<213>  Agrobacterium tumefaciens

<400>  14
gtgaccatgg aactccagcg tccgcgcgaa gcggctgccc tcactttgtc cttgctggac      60

ctgaccaatc ttagggaaga ctgcacgccg cagcagatcg caaccctctg ccagcgggcg     120

catacggagt ttggcaacac cgctgccatt tgcatctggc cgcgtttcgt cgcgcaggcc     180

cgagcggcgt tcggaaaaga ccacacgatt cgcatcgcaa cggtcgtgaa tttcccctcc     240

ggcgatctcg atgtcgcgac cgtggttgcg gaaacggaag ctgcaatcgg cgatggcgcc     300

gacgaaatcg atctggtcat tccctatcgt aaattcatgg caggcgatga atcggcggtg     360

gccgaaatga tcgcggccgt gcgtaaggct tgcgcggcac ctgtgttgct caaggtcatt     420

cttgagaccg gtgagctgaa ggacaaggcc ctgatccgcc gtgcctcgga aatcgccatt     480

gccgaagggg cggatttcat caagacctcg accggcaagg tcgccgtcaa tgccacgctg     540

gaagcggccg atatcatgct gcaggcgatc cgggacagca aaaagaaggt gggcttcaag     600

ccggccggcg gcatcggcac ggtggaggac gcgacactat acctgcggct ggcggaaacc     660

atcatggcgc ccaactgggc catgccgtcg accttccgtt tcggtgcctc gggcgtcctc     720

gatgatgtgc tgaacgtgct ggccggcggc gaaccggcca aggccgccag cgggtattga     780

<210>  15
<211>  259
<212>  PRT
<213>  Agrobacterium tumefaciens

<400>  15

Met Thr Met Glu Leu Gln Arg Pro Arg Glu Ala Ala Ala Leu Thr Leu
1               5                   10                  15

Ser Leu Leu Asp Leu Thr Asn Leu Arg Glu Asp Cys Thr Pro Gln Gln
            20                  25                  30

Ile Ala Thr Leu Cys Gln Arg Ala His Thr Glu Phe Gly Asn Thr Ala
        35                  40                  45

Ala Ile Cys Ile Trp Pro Arg Phe Val Ala Gln Ala Arg Ala Ala Phe
    50                  55                  60

Gly Lys Asp His Thr Ile Arg Ile Ala Thr Val Val Asn Phe Pro Ser
65                  70                  75                  80

Gly Asp Leu Asp Val Ala Thr Val Val Ala Glu Thr Glu Ala Ala Ile
                85                  90                  95

Gly Asp Gly Ala Asp Glu Ile Asp Leu Val Ile Pro Tyr Arg Lys Phe
            100                 105                 110

Met Ala Gly Asp Glu Ser Ala Val Ala Glu Met Ile Ala Ala Val Arg
        115                 120                 125

Lys Ala Cys Ala Ala Pro Val Leu Leu Lys Val Ile Leu Glu Thr Gly
    130                 135                 140

Glu Leu Lys Asp Lys Ala Leu Ile Arg Arg Ala Ser Glu Ile Ala Ile
145                 150                 155                 160

Ala Glu Gly Ala Asp Phe Ile Lys Thr Ser Thr Gly Lys Val Ala Val
                165                 170                 175

Asn Ala Thr Leu Glu Ala Ala Asp Ile Met Leu Gln Ala Ile Arg Asp
            180                 185                 190

Ser Lys Lys Lys Val Gly Phe Lys Pro Ala Gly Gly Ile Gly Thr Val
        195                 200                 205

Glu Asp Ala Thr Leu Tyr Leu Arg Leu Ala Glu Thr Ile Met Ala Pro
    210                 215                 220

Asn Trp Ala Met Pro Ser Thr Phe Arg Phe Gly Ala Ser Gly Val Leu
225                 230                 235                 240

Asp Asp Val Leu Asn Val Leu Ala Gly Gly Glu Pro Ala Lys Ala Ala
                245                 250                 255

Ser Gly Tyr

<210>  16
<211>  681
<212>  DNA
<213>  Anabaena variabilis

<400>  16
atggcagcag actatccgaa cattgatatt gcgccattta tcgatcacgc cctgttaacg      60

ccaacggcta ctccagagca ggttgaccaa tggtgtgaac aagcagacag atataatttt     120

gcgtcggttt gtttgtatcc tacttatgta aaacaagcag cagaatttct ccacggcaag     180

aaacctaagg tttgtacggt aattggtttt cctactgggg ctacgactcg ctcagtcaag     240

ttgtatgagg cactggaagc ggtggagaat ggagccacag agctagatgt agtcatcaat     300

ttgggctgct tgaaatctgg taatacggaa gcagtacacc gggaaattgc cgaaatttgc     360

gaagagactg gacaagtagt taaagtaatt ttggaaacaa acttactgac ggatgcagaa     420

aaaaaaatcg cggccgatat agcaatggat gcaggagcca cattcttaaa aaccaataca     480

ggttggaatg gcggtgctac agtggcagat gtgcggcttt taaaagaaat cacacgggaa     540

agggtgggta taaaggcatc tggtgggatt cgcaccctca atcaagcctt agacttaata     600

ttagcgggtg cgactagatt aggtacgtct cgtggtatcg atttaatcca ccagcgagat     660

aacccggaaa aagttgaata g                                               681

<210>  17
<211>  226
<212>  PRT
<213>  Anabaena variabilis

<400>  17

Met Ala Ala Asp Tyr Pro Asn Ile Asp Ile Ala Pro Phe Ile Asp His
1               5                   10                  15

Ala Leu Leu Thr Pro Thr Ala Thr Pro Glu Gln Val Asp Gln Trp Cys
            20                  25                  30

Glu Gln Ala Asp Arg Tyr Asn Phe Ala Ser Val Cys Leu Tyr Pro Thr
        35                  40                  45

Tyr Val Lys Gln Ala Ala Glu Phe Leu His Gly Lys Lys Pro Lys Val
    50                  55                  60

Cys Thr Val Ile Gly Phe Pro Thr Gly Ala Thr Thr Arg Ser Val Lys
65                  70                  75                  80

Leu Tyr Glu Ala Leu Glu Ala Val Glu Asn Gly Ala Thr Glu Leu Asp
                85                  90                  95

Val Val Ile Asn Leu Gly Cys Leu Lys Ser Gly Asn Thr Glu Ala Val
            100                 105                 110

His Arg Glu Ile Ala Glu Ile Cys Glu Glu Thr Gly Gln Val Val Lys

        115                 120                 125

Val Ile Leu Glu Thr Asn Leu Leu Thr Asp Ala Glu Lys Lys Ile Ala
    130                 135                 140

Ala Asp Ile Ala Met Asp Ala Gly Ala Thr Phe Leu Lys Thr Asn Thr
145                 150                 155                 160

Gly Trp Asn Gly Gly Ala Thr Val Ala Asp Val Arg Leu Leu Lys Glu
                165                 170                 175

Ile Thr Arg Glu Arg Val Gly Ile Lys Ala Ser Gly Gly Ile Arg Thr
            180                 185                 190

Leu Asn Gln Ala Leu Asp Leu Ile Leu Ala Gly Ala Thr Arg Leu Gly
        195                 200                 205

Thr Ser Arg Gly Ile Asp Leu Ile His Gln Arg Asp Asn Pro Glu Lys
    210                 215                 220

Val Glu
225

<210>  18
<211>  681
<212>  DNA
<213>  Pseudomonas syringae

<400>  18
atgaattcgc tcgaacccgc tgcactggcc caggccatcg atcacacctt gttggcggcg      60

gatgccagcc gagagcagat tgccacgctt tgcgcagaag cccgggaaca cggcttctac     120

tcggtgtgcg tgaactccag ccaggtgcct tttgccgccc gacaactggc cgggtctgcc     180

gtgaaggtct gtgcggtggt gggctttccg ctgggcgccg ggctgagtgc cagcaaggcg     240

tcggaagcag ccctgacgat cgccgccggg gctcaggaaa tcgacatggt gctgaacatc     300

ggctggctca aggacggtct gttcgatgag gtccgcgacg atatcgccgc ggtgctgcaa     360

gcctgtggca aggtgccgct caaggtgatc ctggaaacct gcctgctcga tgaggcgcag     420

aaggtgcgcg cctgcgagat ctgccgcgag ctgggcgtgg cattcgtcaa gacctccact     480

ggcttcagcc gcagcggcgc gacgctcgag gatgtggcgc tgatgcgccg tgtggtaggc     540

cctgacatcg gcgtcaaggc gtctggcggg gtgcgtgacg tggccacggc cagagcgatg     600

atcgaagctg gcgcaacgcg cctgggcacc agttccggga ttgcgatcgt gaccggcgca     660

ggtacggggg cgggttattg a                                               681

<210>  19
<211>  226
<212>  PRT
<213>  Pseudomonas syringae

<400>  19

Met Asn Ser Leu Glu Pro Ala Ala Leu Ala Gln Ala Ile Asp His Thr
1               5                   10                  15

Leu Leu Ala Ala Asp Ala Ser Arg Glu Gln Ile Ala Thr Leu Cys Ala
            20                  25                  30

Glu Ala Arg Glu His Gly Phe Tyr Ser Val Cys Val Asn Ser Ser Gln
        35                  40                  45

Val Pro Phe Ala Ala Arg Gln Leu Ala Gly Ser Ala Val Lys Val Cys
    50                  55                  60

Ala Val Val Gly Phe Pro Leu Gly Ala Gly Leu Ser Ala Ser Lys Ala
65                  70                  75                  80

Ser Glu Ala Ala Leu Thr Ile Ala Ala Gly Ala Gln Glu Ile Asp Met
                85                  90                  95

Val Leu Asn Ile Gly Trp Leu Lys Asp Gly Leu Phe Asp Glu Val Arg
            100                 105                 110

Asp Asp Ile Ala Ala Val Leu Gln Ala Cys Gly Lys Val Pro Leu Lys
        115                 120                 125

Val Ile Leu Glu Thr Cys Leu Leu Asp Glu Ala Gln Lys Val Arg Ala
    130                 135                 140

Cys Glu Ile Cys Arg Glu Leu Gly Val Ala Phe Val Lys Thr Ser Thr
145                 150                 155                 160

Gly Phe Ser Arg Ser Gly Ala Thr Leu Glu Asp Val Ala Leu Met Arg
                165                 170                 175

Arg Val Val Gly Pro Asp Ile Gly Val Lys Ala Ser Gly Gly Val Arg
            180                 185                 190

Asp Val Ala Thr Ala Arg Ala Met Ile Glu Ala Gly Ala Thr Arg Leu
        195                 200                 205

Gly Thr Ser Ser Gly Ile Ala Ile Val Thr Gly Ala Gly Thr Gly Ala
    210                 215                 220

Gly Tyr
225

<210>  20
<211>  669
<212>  DNA
<213>  Erwinia carotovora

<400>  20
atgactgact acgcacgcta tatcgaccac accctgctgg cagcaaatgc caccgaacag      60

caaatcgtca cgctgtgcga tgaagcgata gcgcaccatt tttatgccgt ctgtgttaat 120

tccggctacg ttcccttagt agctgaaaaa ttgaaaggtt cagcggtaca ggtgtgctcg 180

gttattggat ttcctctcgg tgcaggcctg acttccagca aagcatttga agcgaaagcc 240

gcgattgatg ccggtgcaca ggaaatcgac atggtgatta acgtcggctg gctgaaaagc 300

gggaagattg atgccgtcaa agcggatatt caggccgtgc gtggggtttg cgccgctata 360

ccgttgaagg taatattgga aacctgtctg cttgatgacg aacagattgt gctggtgtgt 420

gaaatgtgtc gtcagttgga tgtcgcgttc gtcaaaacgt ctaccggttt cagcaccgac 480

ggcgcacgcg aagaacacgt tcgactgatg cgtagcactg tcggcagcga gatgggtgta 540

aaagcctccg gcgcggttcg cgatcgcgaa acggcacaac gaatgattga agcaggcgcc 600

acgcgtattg gcaccagttc aggcgttgct atcgtttctg atgacgctgc cgcagcaggg 660

aactactaa 669

<210> 21
<211> 222
<212> PRT
<213> Erwinia carotovora

<400> 21

Met Thr Asp Tyr Ala Arg Tyr Ile Asp His Thr Leu Leu Ala Ala Asn
1               5                   10                  15

Ala Thr Glu Gln Gln Ile Val Thr Leu Cys Asp Glu Ala Ile Ala His
            20                  25                  30

His Phe Tyr Ala Val Cys Val Asn Ser Gly Tyr Val Pro Leu Val Ala
        35                  40                  45

Glu Lys Leu Lys Gly Ser Ala Val Gln Val Cys Ser Val Ile Gly Phe
    50                  55                  60

Pro Leu Gly Ala Gly Leu Thr Ser Ser Lys Ala Phe Glu Ala Lys Ala
65                  70                  75                  80

Ala Ile Asp Ala Gly Ala Gln Glu Ile Asp Met Val Ile Asn Val Gly
                85                  90                  95

Trp Leu Lys Ser Gly Lys Ile Asp Ala Val Lys Ala Asp Ile Gln Ala
            100                 105                 110

Val Arg Gly Val Cys Ala Ala Ile Pro Leu Lys Val Ile Leu Glu Thr
        115                 120                 125

Cys Leu Leu Asp Asp Glu Gln Ile Val Leu Val Cys Glu Met Cys Arg
    130                 135                 140

Gln Leu Asp Val Ala Phe Val Lys Thr Ser Thr Gly Phe Ser Thr Asp

145                 150                 155                 160

Gly Ala Arg Glu Glu His Val Arg Leu Met Arg Ser Thr Val Gly Ser
                165                 170                 175

Glu Met Gly Val Lys Ala Ser Gly Ala Val Arg Asp Arg Glu Thr Ala
            180                 185                 190

Gln Arg Met Ile Glu Ala Gly Ala Thr Arg Ile Gly Thr Ser Ser Gly
        195                 200                 205

Val Ala Ile Val Ser Asp Asp Ala Ala Ala Ala Gly Asn Tyr
    210                 215                 220

<210>  22
<211>  684
<212>  DNA
<213>  Desulfotalea psychrophila

<400>  22
atgaatacaa tcattagccc gaaagaaatt gccttgtata ttgatcacac tctcctcaaa      60

cctgaggcaa gccctgcagc tattcgtacc ctatgcgcag aagctcgtga gtactctttc     120

aagactgtat gcgtcaactc ttgctatgtc cctctctgtg tggaagaact tcaagcttgc     180

cccgttgatg tttgctcggt ggtggggttc ccacttgggg ctatgctgag ttcggcaaag     240

gcctacgagg caaaacttgc agtggcagcc ggggccgacg aaattgatat ggttatcaat     300

attggtctct tgaaggcagg agaacttgaa gctgttcggg cagatattga aacagttttt     360

gccgcctgtg gagaggcaga ccttaaggtg atcattgaga caggcctgct cagcgatgcg     420

gagaaaaaaa gcgtctgtca gatatgcaag gaagttggtg tcgcctttgt taagacctcc     480

acgggttttg gtcatggtgg cgcaaccgtt gccgatgtag aacttatgcg tgctgttgtt     540

ggtgagagat gtaaggttaa ggcctctggc ggggtacgca accttgccga tgcccgcgcc     600

ctgatagcgg caggagccaa tagaattggg gcaagtgccg gtatcgcaat tgtcaatgga     660

gaagaggtcc ccccttctcg ttaa                                            684

<210>  23
<211>  227
<212>  PRT
<213>  Desulfotalea psychrophila

<400>  23

Met Asn Thr Ile Ile Ser Pro Lys Glu Ile Ala Leu Tyr Ile Asp His
1               5                   10                  15

Thr Leu Leu Lys Pro Glu Ala Ser Pro Ala Ala Ile Arg Thr Leu Cys
            20                  25                  30

Ala Glu Ala Arg Glu Tyr Ser Phe Lys Thr Val Cys Val Asn Ser Cys
        35                  40                  45

Tyr Val Pro Leu Cys Val Glu Glu Leu Gln Ala Cys Pro Val Asp Val
    50                  55                  60

Cys Ser Val Val Gly Phe Pro Leu Gly Ala Met Leu Ser Ser Ala Lys
65                  70                  75                  80

Ala Tyr Glu Ala Lys Leu Ala Val Ala Ala Gly Ala Asp Glu Ile Asp
                85                  90                  95

Met Val Ile Asn Ile Gly Leu Leu Lys Ala Gly Glu Leu Glu Ala Val
            100                 105                 110

Arg Ala Asp Ile Glu Thr Val Phe Ala Ala Cys Gly Glu Ala Asp Leu
        115                 120                 125

Lys Val Ile Ile Glu Thr Gly Leu Leu Ser Asp Ala Glu Lys Lys Ser
    130                 135                 140

Val Cys Gln Ile Cys Lys Glu Val Gly Val Ala Phe Val Lys Thr Ser
145                 150                 155                 160

Thr Gly Phe Gly His Gly Gly Ala Thr Val Ala Asp Val Glu Leu Met
                165                 170                 175

Arg Ala Val Val Gly Glu Arg Cys Lys Val Lys Ala Ser Gly Gly Val
            180                 185                 190

Arg Asn Leu Ala Asp Ala Arg Ala Leu Ile Ala Ala Gly Ala Asn Arg
        195                 200                 205

Ile Gly Ala Ser Ala Gly Ile Ala Ile Val Asn Gly Glu Glu Val Pro
    210                 215                 220

Pro Ser Arg
225

<210>  24
<211>  636
<212>  DNA
<213>  Bacillus subtilis

<400>  24
atgtcattag ccaacataat tgatcataca gctttgaaac cgcatacaca aaaagcggac      60

attctaaaac taattgaaga agcgaaaaca tacaaatttg cttcagtatg tgtcaatccg     120

acatgggtgg agcttgctgc aaaagagctt aagggaactg gagtcgacgt ttgtacggtc     180

atcggcttcc cgctcggtgc caatacaact gaaacaaaag cgttcgaaac aaaagacgcc     240

atttcaaaag gcgccactga agtggatatg gtcattaata ttgccgcttt aaaagacaag     300

gaagacgatg tggtggaagc tgatatccgc ggtgtagtgg aagctgtagc cggaaaagcg     360

cttgtcaaag tcattatcga aacgtgcctt ctgactgatg aagaaaaaga acgtgcatgc     420

cgtttagcgg tgtctgcggg agcggatttc gtaaaaacat caacaggctt ttctacaggc 480

ggcgcaacga aggaagatat cgccttaatg cgcaaaacag tagggcctga tatcggcgtg 540

aaagcatctg gcggcgtcag aacgaaagaa gatgtagaca caatggtaga ggccggagca 600

agccgaattg cgccagcgca ggcgtttcta tcgtaa 636

<210> 25
<211> 211
<212> PRT
<213> Bacillus subtilis

<400> 25

Met Ser Leu Ala Asn Ile Ile Asp His Thr Ala Leu Lys Pro His Thr
1               5                   10                  15

Gln Lys Ala Asp Ile Leu Lys Leu Ile Glu Glu Ala Lys Thr Tyr Lys
            20                  25                  30

Phe Ala Ser Val Cys Val Asn Pro Thr Trp Val Glu Leu Ala Ala Lys
        35                  40                  45

Glu Leu Lys Gly Thr Gly Val Asp Val Cys Thr Val Ile Gly Phe Pro
    50                  55                  60

Leu Gly Ala Asn Thr Thr Glu Thr Lys Ala Phe Glu Thr Lys Asp Ala
65                  70                  75                  80

Ile Ser Lys Gly Ala Thr Glu Val Asp Met Val Ile Asn Ile Ala Ala
                85                  90                  95

Leu Lys Asp Lys Glu Asp Asp Val Val Glu Ala Asp Ile Arg Gly Val
            100                 105                 110

Val Glu Ala Val Ala Gly Lys Ala Leu Val Lys Val Ile Ile Glu Thr
        115                 120                 125

Cys Leu Leu Thr Asp Glu Glu Lys Glu Arg Ala Cys Arg Leu Ala Val
    130                 135                 140

Ser Ala Gly Ala Asp Phe Val Lys Thr Ser Thr Gly Phe Ser Thr Gly
145                 150                 155                 160

Gly Ala Thr Lys Glu Asp Ile Ala Leu Met Arg Lys Thr Val Gly Pro
                165                 170                 175

Asp Ile Gly Val Lys Ala Ser Gly Gly Val Arg Thr Lys Glu Asp Val
            180                 185                 190

Asp Thr Met Val Glu Ala Gly Ala Ser Arg Ile Ala Pro Ala Gln Ala
        195                 200                 205

Phe Leu Ser
    210

<210>  26
<211>  672
<212>  DNA
<213>  Listeria innocua

<400>  26
atgacaattg ccaaaatgat cgaccacact gctttaaaac cagacacaac gaaagaacaa      60

attttaacat taacaaaaga agcaagagaa tatggttttg cttccgtatg cgtgaatcca     120

acttgggtga aattatccgc tgaacagctt tcaggagcag aatccgttgt atgtacagtt     180

atcggtttcc cacttggagc aaatacacca gaagtaaaag cttttgaagt gaaaaatgcc     240

atcgaaaacg gcgctaaaga agtggatatg gttattaata tcggcgcatt aaaagacaaa     300

gacgatgaat tagtagaacg tgatattcgt gctgtagttg atgctgccaa agggaaagca     360

ttagtaaaag taattattga aacttgccta ttaacagacg aagaaaaagt tcgcgcatgt     420

gaaatcgctg taaaagcagg aacagacttc gttaaaacat ccactggatt ctccacaggt     480

ggcgcaactg ccgaagatat tgctttaatg cgtaaaactg taggaccaaa catcggcgta     540

aaagcatctg gcggagttcg tacaaaagaa gacgtagaaa aaatgattga agcaggtgca     600

actcgtatcg gcgcaagtgc aggtgtcgca attgtttccg gcgaaaaacc agctaaacca     660

gataattact aa                                                         672

<210>  27
<211>  223
<212>  PRT
<213>  Listeria innocua

<400>  27

Met Thr Ile Ala Lys Met Ile Asp His Thr Ala Leu Lys Pro Asp Thr
1               5                   10                  15

Thr Lys Glu Gln Ile Leu Thr Leu Thr Lys Glu Ala Arg Glu Tyr Gly
            20                  25                  30

Phe Ala Ser Val Cys Val Asn Pro Thr Trp Val Lys Leu Ser Ala Glu
        35                  40                  45

Gln Leu Ser Gly Ala Glu Ser Val Val Cys Thr Val Ile Gly Phe Pro
    50                  55                  60

Leu Gly Ala Asn Thr Pro Glu Val Lys Ala Phe Glu Val Lys Asn Ala
65                  70                  75                  80

Ile Glu Asn Gly Ala Lys Glu Val Asp Met Val Ile Asn Ile Gly Ala
                85                  90                  95

Leu Lys Asp Lys Asp Asp Glu Leu Val Glu Arg Asp Ile Arg Ala Val

            100                 105                 110

Val Asp Ala Ala Lys Gly Lys Ala Leu Val Lys Val Ile Ile Glu Thr
        115                 120                 125

Cys Leu Leu Thr Asp Glu Glu Lys Val Arg Ala Cys Glu Ile Ala Val
    130                 135                 140

Lys Ala Gly Thr Asp Phe Val Lys Thr Ser Thr Gly Phe Ser Thr Gly
145                 150                 155                 160

Gly Ala Thr Ala Glu Asp Ile Ala Leu Met Arg Lys Thr Val Gly Pro
                165                 170                 175

Asn Ile Gly Val Lys Ala Ser Gly Gly Val Arg Thr Lys Glu Asp Val
            180                 185                 190

Glu Lys Met Ile Glu Ala Gly Ala Thr Arg Ile Gly Ala Ser Ala Gly
        195                 200                 205

Val Ala Ile Val Ser Gly Glu Lys Pro Ala Lys Pro Asp Asn Tyr
    210                 215                 220

<210> 28
<211> 648
<212> DNA
<213> Clostridium acetobutylicum

<400> 28
atgaatattg ctaaaattat agatcataca gcattaaagc cagatacaac aaaggagcag 60

atactaaaac taatagaaga agctaaacaa aataactttg catcagtttg tgtaaatcca 120

aagtgggtta aagaggcaag ctgtgcatta aaggacagca gtgttaaagt gtgtactgta 180

atagggtttc ctcttggagc taatacaact gctacaaaag tatttgaaac acaagatgct 240

attaaaaatg gtgcagaaga agtagatatg gttgtttcta taggagaatt aaaagataaa 300

aatgatgatt atgtagaaaa agatatagaa gaagttgtta aggcagctag tggaaaggcc 360

ttagttaaag taattattga aacttgtctt cttaccgaag aagagaagat aagagcgtgt 420

aaactagcta aaaaagcagg tgcagatttt gttaaaacat caacagggtt ttcaacagga 480

ggggctaagg cagaagatat taaattaatg agaaaaacag ttggagctgg tatgggagtt 540

aaggcctcag gtggtattca tacaagagaa gaagcaatta aacttataga agctggagct 600

acacgtattg gagctagtgc aagtatagat ataatttcag aaaattaa 648

<210> 29
<211> 215
<212> PRT
<213> Clostridium acetobutylicum

<400> 29

Met Asn Ile Ala Lys Ile Ile Asp His Thr Ala Leu Lys Pro Asp Thr

1               5                   10                  15

Thr Lys Glu Gln Ile Leu Lys Leu Ile Glu Glu Ala Lys Gln Asn Asn
            20                  25                  30

Phe Ala Ser Val Cys Val Asn Pro Lys Trp Val Lys Glu Ala Ser Cys
        35                  40                  45

Ala Leu Lys Asp Ser Ser Val Lys Val Cys Thr Val Ile Gly Phe Pro
    50                  55                  60

Leu Gly Ala Asn Thr Thr Ala Thr Lys Val Phe Glu Thr Gln Asp Ala
65                  70                  75                  80

Ile Lys Asn Gly Ala Glu Glu Val Asp Met Val Val Ser Ile Gly Glu
                85                  90                  95

Leu Lys Asp Lys Asn Asp Asp Tyr Val Glu Lys Asp Ile Glu Glu Val
            100                 105                 110

Val Lys Ala Ala Ser Gly Lys Ala Leu Val Lys Val Ile Ile Glu Thr
        115                 120                 125

Cys Leu Leu Thr Glu Glu Glu Lys Ile Arg Ala Cys Lys Leu Ala Lys
    130                 135                 140

Lys Ala Gly Ala Asp Phe Val Lys Thr Ser Thr Gly Phe Ser Thr Gly
145                 150                 155                 160

Gly Ala Lys Ala Glu Asp Ile Lys Leu Met Arg Lys Thr Val Gly Ala
                165                 170                 175

Gly Met Gly Val Lys Ala Ser Gly Gly Ile His Thr Arg Glu Glu Ala
            180                 185                 190

Ile Lys Leu Ile Glu Ala Gly Ala Thr Arg Ile Gly Ala Ser Ala Ser
        195                 200                 205

Ile Asp Ile Ile Ser Glu Asn
    210                 215

<210>  30
<211>  822
<212>  DNA
<213>  Geobacillus kaustophilus

<400>  30
atggagctca taacccagcc tagttgctgg gttttttctg tctttttccg gagacaatac      60

ggatggctag tttttgtcga gggagcatgg tatgatggta gacgtcagac tttccatctt     120

gacggaaatg gcagaaaggg gtttctccga atgacgatga atatcgcgaa aatgatcgat     180

catacgctgc tcaaaccgga agcgacagaa caacaaatcg tgcaactgtg cacggaagca     240

aagcaatacg gctttgcttc cgtgtgcgtc aacccaacgt gggtgaaaac ggcggcgcgc 300

gagctttccg gcacggatgt ccgcgtctgc acggtcatcg gctttccact tggggcaacg 360

acgccggaaa caaaggcgtt tgaaacaacg aacgccatcg aaaacggcgc tcgcgaagtc 420

gacatggtga tcaacatcgg cgcgttaaaa agcgggcaag acgagcttgt cgagcgcgac 480

attcgtgcgg ttgtcgaagc ggcggctggc agggcgcttg tcaaagtgat cgttgaaacg 540

gcgcttttga ccgatgagga aaaagtgcgc gcctgccagc tcgcagtgaa agccggcgct 600

gattatgtga aaacgtcgac cgggttttcc ggcggaggtg cgacggtgga ggatgtggcg 660

ttgatgcgga aaacggtcgg cgacagagca ggcgtcaaag catcaggcgg cgtccgtgac 720

tggaaaaccg ctgaggcgat gatcaacgcc ggcgcgacgc gcatcggcac aagctctggg 780

gtggcgatcg tcaccggcgg gacgggccgc gctgactact aa 822

<210> 31
<211> 273
<212> PRT
<213> Geobacillus kaustophilus

<400> 31

Met Glu Leu Ile Thr Gln Pro Ser Cys Trp Val Phe Ser Val Phe Phe
1               5                   10                  15

Arg Arg Gln Tyr Gly Trp Leu Val Phe Val Glu Gly Ala Trp Tyr Asp
            20                  25                  30

Gly Arg Arg Gln Thr Phe His Leu Asp Gly Asn Gly Arg Lys Gly Phe
        35                  40                  45

Leu Arg Met Thr Met Asn Ile Ala Lys Met Ile Asp His Thr Leu Leu
    50                  55                  60

Lys Pro Glu Ala Thr Glu Gln Gln Ile Val Gln Leu Cys Thr Glu Ala
65                  70                  75                  80

Lys Gln Tyr Gly Phe Ala Ser Val Cys Val Asn Pro Thr Trp Val Lys
                85                  90                  95

Thr Ala Ala Arg Glu Leu Ser Gly Thr Asp Val Arg Val Cys Thr Val
            100                 105                 110

Ile Gly Phe Pro Leu Gly Ala Thr Thr Pro Glu Thr Lys Ala Phe Glu
        115                 120                 125

Thr Thr Asn Ala Ile Glu Asn Gly Ala Arg Glu Val Asp Met Val Ile
    130                 135                 140

Asn Ile Gly Ala Leu Lys Ser Gly Gln Asp Glu Leu Val Glu Arg Asp
145                 150                 155                 160

Ile Arg Ala Val Val Glu Ala Ala Ala Gly Arg Ala Leu Val Lys Val
                165                 170                 175

Ile Val Glu Thr Ala Leu Leu Thr Asp Glu Glu Lys Val Arg Ala Cys
            180                 185                 190

Gln Leu Ala Val Lys Ala Gly Ala Asp Tyr Val Lys Thr Ser Thr Gly
        195                 200                 205

Phe Ser Gly Gly Gly Ala Thr Val Glu Asp Val Ala Leu Met Arg Lys
    210                 215                 220

Thr Val Gly Asp Arg Ala Gly Val Lys Ala Ser Gly Gly Val Arg Asp
225                 230                 235                 240

Trp Lys Thr Ala Glu Ala Met Ile Asn Ala Gly Ala Thr Arg Ile Gly
                245                 250                 255

Thr Ser Ser Gly Val Ala Ile Val Thr Gly Gly Thr Gly Arg Ala Asp
            260                 265                 270

Tyr

<210>  32
<211>  675
<212>  DNA
<213>  Bacillus halodurans

<400>  32
atgtcacgtt cgattgcaca aatgattgat catacgctac ttaaaccaaa tacaacagaa      60

gaccaaattg taaagctctg tgaggaagca aaggaatatt catttgcatc tgtttgtgtg     120

aatcctactt gggtcgctct tgctgcgcag ttgctaaaag atgcacctga tgtgaaagta     180

tgtacagtta tcggctttcc gttaggggca acgactccgg aagtgaaagc gtttgaaacg     240

actaatgcca ttgaaaatgg agcgacagaa gtggacatgg tcattaacat tggagcgtta     300

aaagataaac aatacgagct tgttggacgc gacattcaag cggttgttaa agcagcagaa     360

gggaaagcat taacgaaagt aatcattgaa acatcgttat taacggagga agagaagaag     420

gctgcgtgtg agcttgccgt aaaagcagga gccgactttg tcaaaacgtc gactggattc     480

tctggcggag gtgctacggc tgaggatatc gcgctcatgc gaaaagtggt cggaccaaat     540

ttaggagtca aagcttctgg aggtgttaga gatctgtccg acgcgaaagc gatgattgat     600

gctggtgcta ctcggattgg tgcgagtgct ggggtggcga ttgttaacgg ggagcgtagc     660

gaagggagtt attaa                                                      675

<210>  33
<211>  224
<212>  PRT
<213>  Bacillus halodurans

<400>  33

Met Ser Arg Ser Ile Ala Gln Met Ile Asp His Thr Leu Leu Lys Pro
1               5                   10                  15

Asn Thr Thr Glu Asp Gln Ile Val Lys Leu Cys Glu Glu Ala Lys Glu
            20                  25                  30

Tyr Ser Phe Ala Ser Val Cys Val Asn Pro Thr Trp Val Ala Leu Ala
        35                  40                  45

Ala Gln Leu Leu Lys Asp Ala Pro Asp Val Lys Val Cys Thr Val Ile
    50                  55                  60

Gly Phe Pro Leu Gly Ala Thr Thr Pro Glu Val Lys Ala Phe Glu Thr
65                  70                  75                  80

Thr Asn Ala Ile Glu Asn Gly Ala Thr Glu Val Asp Met Val Ile Asn
                85                  90                  95

Ile Gly Ala Leu Lys Asp Lys Gln Tyr Glu Leu Val Gly Arg Asp Ile
            100                 105                 110

Gln Ala Val Val Lys Ala Ala Glu Gly Lys Ala Leu Thr Lys Val Ile
        115                 120                 125

Ile Glu Thr Ser Leu Leu Thr Glu Glu Glu Lys Lys Ala Ala Cys Glu
    130                 135                 140

Leu Ala Val Lys Ala Gly Ala Asp Phe Val Lys Thr Ser Thr Gly Phe
145                 150                 155                 160

Ser Gly Gly Gly Ala Thr Ala Glu Asp Ile Ala Leu Met Arg Lys Val
                165                 170                 175

Val Gly Pro Asn Leu Gly Val Lys Ala Ser Gly Gly Val Arg Asp Leu
            180                 185                 190

Ser Asp Ala Lys Ala Met Ile Asp Ala Gly Ala Thr Arg Ile Gly Ala
        195                 200                 205

Ser Ala Gly Val Ala Ile Val Asn Gly Glu Arg Ser Glu Gly Ser Tyr
    210                 215                 220

<210>  34
<211>  672
<212>  DNA
<213>  Bacillus licheniformis

<400>  34
atgacaaaac aaattgcgcg aatgatcgat cacactgcat tgaagccaga taccgtcaaa      60

tccgaaatcg aagcgctttg caaagaagcg cgtgtttacg gttttgcctc cgtttgtgtc     120

aacccttgct gggtgaagct ttgcgccgag cttcttaaag agtcagaggt gaaagtatgt 180

acagttatcg gctttccttt aggtgcagcg tctccggaaa caaaagcctt tgaaaccagg 240

caggcaattg cagacggtgc cggtgaagtt gatatggtga tcaacatcgg tgcactaaaa 300

gaccgcgata cgggaacagt ggaacatgac atcagggcgg tgacagacgc ggccgacggc 360

aaagctcttg taaaagtcat catagagacg tcgcttttga cggatgaaga aaaaaggctg 420

gcttgtgaac tggccgtaaa agcaggcgcc gactttgtca aaacatcgac cggtttttcc 480

ggcggcggtg cgacagtccg ggatataaaa ctgatgcggg aagctgtcgg acctgatatc 540

ggcgttaaag cttcaggtgg cgtccgcgat aaggaaagcg cacttgccat gattgaagcc 600

ggagcgacga gaatcggagc gagcgccggc gtgtcgattg tcaaagggtt aacagcggat 660

gaagactact aa 672

<210> 35
<211> 223
<212> PRT
<213> Bacillus licheniformis

<400> 35

Met Thr Lys Gln Ile Ala Arg Met Ile Asp His Thr Ala Leu Lys Pro
1               5                   10                  15

Asp Thr Val Lys Ser Glu Ile Glu Ala Leu Cys Lys Glu Ala Arg Val
            20                  25                  30

Tyr Gly Phe Ala Ser Val Cys Val Asn Pro Cys Trp Val Lys Leu Cys
        35                  40                  45

Ala Glu Leu Leu Lys Glu Ser Glu Val Lys Val Cys Thr Val Ile Gly
    50                  55                  60

Phe Pro Leu Gly Ala Ala Ser Pro Glu Thr Lys Ala Phe Glu Thr Arg
65                  70                  75                  80

Gln Ala Ile Ala Asp Gly Ala Gly Glu Val Asp Met Val Ile Asn Ile
                85                  90                  95

Gly Ala Leu Lys Asp Arg Asp Thr Gly Thr Val Glu His Asp Ile Arg
            100                 105                 110

Ala Val Thr Asp Ala Ala Asp Gly Lys Ala Leu Val Lys Val Ile Ile
        115                 120                 125

Glu Thr Ser Leu Leu Thr Asp Glu Glu Lys Arg Leu Ala Cys Glu Leu
    130                 135                 140

Ala Val Lys Ala Gly Ala Asp Phe Val Lys Thr Ser Thr Gly Phe Ser
145                 150                 155                 160

Gly Gly Gly Ala Thr Val Arg Asp Ile Lys Leu Met Arg Glu Ala Val
                165                 170                 175

Gly Pro Asp Ile Gly Val Lys Ala Ser Gly Gly Val Arg Asp Lys Glu
            180                 185                 190

Ser Ala Leu Ala Met Ile Glu Ala Gly Ala Thr Arg Ile Gly Ala Ser
        195                 200                 205

Ala Gly Val Ser Ile Val Lys Gly Leu Thr Ala Asp Glu Asp Tyr
    210                 215                 220

<210>  36
<211>  663
<212>  DNA
<213>  Streptococcus mutans

<400>  36
atgaaaatca atcaatatat tgaccatact ttattaaaac cagaaagtag gcaagatcag      60

attgataaac tgattcgaga agctaagaca tataattttg ccagtgtctg tatcaatcca     120

acttgggttt cttatgcggc taaagctctt gaaggaacag acattaaagt ttgtactgtt     180

attggttttc ctttaggagc aacgactagt gctgtaaaag cctttgaaac caaggatgct     240

attagtcatg gagctgacga agttgatatg gttatcaata ttggtcaagc taaatctggt     300

cattttgctt ttgttgaaga agatattcgg gcagttgttg aagccagtgg tgacaaattg     360

gtgaaagtta ttattgaaac ttgtctcctt acagataaag aaaaaattaa agcttgtcaa     420

gctgcagtag cagcaggtgc tgatttcgtt aaaacatcaa ctggtttttc aactgctgga     480

gctaggttag atgatgttcg tcttatgcgt caaacggtag gacctgatgt tggagtaaag     540

gcggcaggag gaacgcgatc tttagaagat gcgcaagctt ttattgaagc aggtgcaaca     600

cgtattggga catctgctgg agttactatt atggaaggaa agcaaacaaa cagtggttat     660

tga                                                                   663

<210>  37
<211>  220
<212>  PRT
<213>  Streptococcus mutans

<400>  37

Met Lys Ile Asn Gln Tyr Ile Asp His Thr Leu Leu Lys Pro Glu Ser
1               5                   10                  15

Arg Gln Asp Gln Ile Asp Lys Leu Ile Arg Glu Ala Lys Thr Tyr Asn
            20                  25                  30

Phe Ala Ser Val Cys Ile Asn Pro Thr Trp Val Ser Tyr Ala Ala Lys
        35                  40                  45

Ala Leu Glu Gly Thr Asp Ile Lys Val Cys Thr Val Ile Gly Phe Pro

50                  55                  60

Leu Gly Ala Thr Thr Ser Ala Val Lys Ala Phe Glu Thr Lys Asp Ala
65                  70                  75                  80

Ile Ser His Gly Ala Asp Glu Val Asp Met Val Ile Asn Ile Gly Gln
                85                  90                  95

Ala Lys Ser Gly His Phe Ala Phe Val Glu Glu Asp Ile Arg Ala Val
            100                 105                 110

Val Glu Ala Ser Gly Asp Lys Leu Val Lys Val Ile Ile Glu Thr Cys
        115                 120                 125

Leu Leu Thr Asp Lys Glu Lys Ile Lys Ala Cys Gln Ala Ala Val Ala
    130                 135                 140

Ala Gly Ala Asp Phe Val Lys Thr Ser Thr Gly Phe Ser Thr Ala Gly
145                 150                 155                 160

Ala Arg Leu Asp Asp Val Arg Leu Met Arg Gln Thr Val Gly Pro Asp
                165                 170                 175

Val Gly Val Lys Ala Ala Gly Gly Thr Arg Ser Leu Glu Asp Ala Gln
            180                 185                 190

Ala Phe Ile Glu Ala Gly Ala Thr Arg Ile Gly Thr Ser Ala Gly Val
        195                 200                 205

Thr Ile Met Glu Gly Lys Gln Thr Asn Ser Gly Tyr
    210                 215                 220

<210>  38
<211>  663
<212>  DNA
<213>  Staphylococcus epidermidis

<400>  38
atgaataaag caaaattgat agaccataca ttattaaaac ctgattcaac aaaggaacaa      60

atagatacta ttataaatga agcaaaagca tatcagttta agtctgtatg tgtgaaccct     120

acacatgtac aatatgcatc tgaacaactt aaaggaacag acgttttagt gtgtactgtt     180

attggatttc cactaggtgc aacaactaca gcggttaaat cttatgaaac aaaagatgcg     240

attaacaatg gtgcccaaga gattgatatg gtgataaata ttggagcact taaggatggc     300

cgttttgatg aagtgcaaaa tgatatcgaa gccgtcgttc aagcagccaa tggtaaaaca     360

gttaaggtaa ttattgagac tgttttatta actgagaaag agaagattaa agcatgtcaa     420

ttatctgaag cggcaggtgc acattttgtt aaaacatcca caggttttgc tggtgggggt     480

gcaacagttg aagatgtaaa attaatgaaa gatactgttg gtgatcgttt agaagtaaaa     540

gcgtcaggcg gcgtgagaaa tctagaagat tttaataata tgattgaagc gggtgctaca     600

cgtattggtg ctagtgccgg tgtgcaaatt attcaaggac ttgaatcaaa tactgattac 660

taa 663

<210> 39
<211> 220
<212> PRT
<213> Staphylococcus epidermidis

<400> 39

Met Asn Lys Ala Lys Leu Ile Asp His Thr Leu Leu Lys Pro Asp Ser
1               5                   10                  15

Thr Lys Glu Gln Ile Asp Thr Ile Ile Asn Glu Ala Lys Ala Tyr Gln
            20                  25                  30

Phe Lys Ser Val Cys Val Asn Pro Thr His Val Gln Tyr Ala Ser Glu
        35                  40                  45

Gln Leu Lys Gly Thr Asp Val Leu Val Cys Thr Val Ile Gly Phe Pro
    50                  55                  60

Leu Gly Ala Thr Thr Thr Ala Val Lys Ser Tyr Glu Thr Lys Asp Ala
65                  70                  75                  80

Ile Asn Asn Gly Ala Gln Glu Ile Asp Met Val Ile Asn Ile Gly Ala
                85                  90                  95

Leu Lys Asp Gly Arg Phe Asp Glu Val Gln Asn Asp Ile Glu Ala Val
            100                 105                 110

Val Gln Ala Ala Asn Gly Lys Thr Val Lys Val Ile Ile Glu Thr Val
        115                 120                 125

Leu Leu Thr Glu Lys Glu Lys Ile Lys Ala Cys Gln Leu Ser Glu Ala
    130                 135                 140

Ala Gly Ala His Phe Val Lys Thr Ser Thr Gly Phe Ala Gly Gly Gly
145                 150                 155                 160

Ala Thr Val Glu Asp Val Lys Leu Met Lys Asp Thr Val Gly Asp Arg
                165                 170                 175

Leu Glu Val Lys Ala Ser Gly Gly Val Arg Asn Leu Glu Asp Phe Asn
            180                 185                 190

Asn Met Ile Glu Ala Gly Ala Thr Arg Ile Gly Ala Ser Ala Gly Val
        195                 200                 205

Gln Ile Ile Gln Gly Leu Glu Ser Asn Thr Asp Tyr
    210                 215                 220

<210>  40
<211>  648
<212>  DNA
<213>  Lactobacillus plantarum

<400>  40
atgaaattga atcgttattt agatcacacg ttattaaaac cggaagcgac tgagcaacaa      60

attgatcagg tagtacggga ggcactcgaa aatcactttt attcagttat ggtcaatcca     120

tactgggtca agcacgtcca tgcgcaactt gctggttcgg atgttgcgac tgcatgcgtg     180

attggtttcc ctctgggcgc gaatacaacc gccattaaag ttgcggaagc caaacaggca     240

attgctgacg gtgtggatga gctggatatg gtcattaata tcggcgaatt gaaaggcgac     300

cactatgatg cagttcaaca agacattgaa agtgtggtaa cagttggaca tacggctgat     360

aaggtcgtca aagtgattat tgaaacggcg ctgttgacgg atggggaaat cgttaaggct     420

agtgaaattg ttgccgatgc acacgctgat tttgtgaaga catcgactgg attttcaacc     480

cgtggtgctt cggttcatga tattagtttg atgaagggtg ccgttcagga tcgaatcggg     540

gtcaaagcat ctgggggaat ccatacacgc gatgaagcat tagcgatgat tgatgctgga     600

gcaacgcgcc tcggtgtatc agcaagtatg gcaattattg gtaagtag                  648

<210>  41
<211>  215
<212>  PRT
<213>  Lactobacillus plantarum

<400>  41

Met Lys Leu Asn Arg Tyr Leu Asp His Thr Leu Leu Lys Pro Glu Ala
1               5                   10                  15

Thr Glu Gln Gln Ile Asp Gln Val Val Arg Glu Ala Leu Glu Asn His
            20                  25                  30

Phe Tyr Ser Val Met Val Asn Pro Tyr Trp Val Lys His Val His Ala
        35                  40                  45

Gln Leu Ala Gly Ser Asp Val Ala Thr Ala Cys Val Ile Gly Phe Pro
    50                  55                  60

Leu Gly Ala Asn Thr Thr Ala Ile Lys Val Ala Glu Ala Lys Gln Ala
65                  70                  75                  80

Ile Ala Asp Gly Val Asp Glu Leu Asp Met Val Ile Asn Ile Gly Glu
                85                  90                  95

Leu Lys Gly Asp His Tyr Asp Ala Val Gln Gln Asp Ile Glu Ser Val
            100                 105                 110

Val Thr Val Gly His Thr Ala Asp Lys Val Val Lys Val Ile Ile Glu
        115                 120                 125

Thr Ala Leu Leu Thr Asp Gly Glu Ile Val Lys Ala Ser Glu Ile Val
    130                 135                 140

Ala Asp Ala His Ala Asp Phe Val Lys Thr Ser Thr Gly Phe Ser Thr
145                 150                 155                 160

Arg Gly Ala Ser Val His Asp Ile Ser Leu Met Lys Gly Ala Val Gln
                165                 170                 175

Asp Arg Ile Gly Val Lys Ala Ser Gly Gly Ile His Thr Arg Asp Glu
            180                 185                 190

Ala Leu Ala Met Ile Asp Ala Gly Ala Thr Arg Leu Gly Val Ser Ala
        195                 200                 205

Ser Met Ala Ile Ile Gly Lys
    210                 215

<210>  42
<211>  728
<212>  DNA
<213>  Lactobacillus acidophilus

<400>  42
cagaggtaaa aattatgaaa tatactttag acgactttgc acgtttaatt gatcacacta      60

acttacacgc tgatgcaact gaagccgata tgaagaagtt atgtgatgaa gcaaagaaat     120

atcattttaa aatggtagct attaatcaag ttcaatccaa gttttgctca gagcaattaa     180

agggaacaga cattgatact ggtgctgcaa ttgcttttcc tttaggacaa caaactattg     240

aatccaaggt atttgatact agggatgcaa ttaagaatgg tgctaatgaa attgattatg     300

tgattaatat tactcaatta aaagctaaag actacgatta tataaagcaa gaaatgcaag     360

agatggttaa tgcttgtcat gaaaatcatg ttccatgtaa agtgattttt gaaaattgct     420

atttaaccaa agatgaaata aaaaaattag ctgagattgc taaagaagta aagcctgact     480

ttattaagac ttctactggc tttggtagtt caggcgctaa ggtagaagac gtaaagctaa     540

tgaaatcaat tgttggcgat gaagtaaaag taaaggctgc cggtggtatt cgtaatagtg     600

atgatttctt agccatggtg cgcgctggtg ctgatagaat tggttgttct gctggagtca     660

aaatttatca agctttaaag tgtagaatga aagacgacca tgtggatagt attgagattg     720

cacgttag                                                              728

<210>  43
<211>  237
<212>  PRT
<213>  Lactobacillus acidophilus

<400>  43

Met Lys Tyr Thr Leu Asp Asp Phe Ala Arg Leu Ile Asp His Thr Asn
1               5                   10                  15

Leu His Ala Asp Ala Thr Glu Ala Asp Met Lys Lys Leu Cys Asp Glu
            20                  25                  30

Ala Lys Lys Tyr His Phe Lys Met Val Ala Ile Asn Gln Val Gln Ser
        35                  40                  45

Lys Phe Cys Ser Glu Gln Leu Lys Gly Thr Asp Ile Asp Thr Gly Ala
    50                  55                  60

Ala Ile Ala Phe Pro Leu Gly Gln Gln Thr Ile Glu Ser Lys Val Phe
65                  70                  75                  80

Asp Thr Arg Asp Ala Ile Lys Asn Gly Ala Asn Glu Ile Asp Tyr Val
                85                  90                  95

Ile Asn Ile Thr Gln Leu Lys Ala Lys Asp Tyr Asp Tyr Ile Lys Gln
            100                 105                 110

Glu Met Gln Glu Met Val Asn Ala Cys His Glu Asn His Val Pro Cys
        115                 120                 125

Lys Val Ile Phe Glu Asn Cys Tyr Leu Thr Lys Asp Glu Ile Lys Lys
    130                 135                 140

Leu Ala Glu Ile Ala Lys Glu Val Lys Pro Asp Phe Ile Lys Thr Ser
145                 150                 155                 160

Thr Gly Phe Gly Ser Ser Gly Ala Lys Val Glu Asp Val Lys Leu Met
                165                 170                 175

Lys Ser Ile Val Gly Asp Glu Val Lys Val Lys Ala Ala Gly Gly Ile
            180                 185                 190

Arg Asn Ser Asp Asp Phe Leu Ala Met Val Arg Ala Gly Ala Asp Arg
        195                 200                 205

Ile Gly Cys Ser Ala Gly Val Lys Ile Tyr Gln Ala Leu Lys Cys Arg
    210                 215                 220

Met Lys Asp Asp His Val Asp Ser Ile Glu Ile Ala Arg
225                 230                 235

<210>  44
<211>  672
<212>  DNA
<213>  Streptococcus pyogenes

<400>  44
gtggaagtaa aagatatttt aaaaacggta gaccatactt tgctagcaac aacagcaacg      60

tggccagaaa tccaaacaat tttagatgat gccatggctt atgaaacagc ttcagcatgt     120

attccagctt cttacgtcaa aaaagcagca gaatacgttt caggtaaatt agctatttgt     180

actgttattg ggttcccaaa tggctatagt acaactgcgg cgaaggtttt tgaatgtcaa     240

gatgctattc aaaatggtgc tgatgaaatt gacatggtca ttaatttgac agacgttaaa     300

aatggggatt ttgatactgt tgaagaagaa attcgtcaaa tcaaagctaa atgtcaagac     360

catatcttaa aagttatcgt tgagacatgt caattaacta aagaagaact tatcgaactt     420

tgtggagttg tcacacgttc aggtgcagac tttattaaaa cctctactgg tttttcgaca     480

gcaggtgcta catttgaaga tgttgaagtg atggcaaaat atgtcggcga aggtgttaaa     540

attaaggcag caggtggaat ctcatcattg gaagatgcta aaacatttat tgctttagga     600

gcttcacgct tgggtactag ccgtatcatc aagattgtta agaacgaagc tacaaaaccc     660

gatagctatt aa                                                         672

<210>  45
<211>  223
<212>  PRT
<213>  Streptococcus pyogenes

<400>  45

Met Glu Val Lys Asp Ile Leu Lys Thr Val Asp His Thr Leu Leu Ala
1               5                   10                  15

Thr Thr Ala Thr Trp Pro Glu Ile Gln Thr Ile Leu Asp Asp Ala Met
            20                  25                  30

Ala Tyr Glu Thr Ala Ser Ala Cys Ile Pro Ala Ser Tyr Val Lys Lys
        35                  40                  45

Ala Ala Glu Tyr Val Ser Gly Lys Leu Ala Ile Cys Thr Val Ile Gly
    50                  55                  60

Phe Pro Asn Gly Tyr Ser Thr Thr Ala Ala Lys Val Phe Glu Cys Gln
65                  70                  75                  80

Asp Ala Ile Gln Asn Gly Ala Asp Glu Ile Asp Met Val Ile Asn Leu
                85                  90                  95

Thr Asp Val Lys Asn Gly Asp Phe Asp Thr Val Glu Glu Glu Ile Arg
            100                 105                 110

Gln Ile Lys Ala Lys Cys Gln Asp His Ile Leu Lys Val Ile Val Glu
        115                 120                 125

Thr Cys Gln Leu Thr Lys Glu Glu Leu Ile Glu Leu Cys Gly Val Val
    130                 135                 140

Thr Arg Ser Gly Ala Asp Phe Ile Lys Thr Ser Thr Gly Phe Ser Thr
145                 150                 155                 160

Ala Gly Ala Thr Phe Glu Asp Val Glu Val Met Ala Lys Tyr Val Gly

165                 170                 175

Glu Gly Val Lys Ile Lys Ala Ala Gly Gly Ile Ser Ser Leu Glu Asp
            180                 185                 190

Ala Lys Thr Phe Ile Ala Leu Gly Ala Ser Arg Leu Gly Thr Ser Arg
        195                 200                 205

Ile Ile Lys Ile Val Lys Asn Glu Ala Thr Lys Pro Asp Ser Tyr
    210                 215                 220

<210>  46
<211>  783
<212>  DNA
<213>  Rhodopirellula baltica

<400>  46
ttgcgcgaca cccgacctcc tgccgcatcg ctatcgcttc acggaaacct gctcaccatg      60

gctgactatc aatatcacga cgtctccaag atgattgacc actcgctgct tccacccaca     120

ctgaccgaag cggacttgga ttccggcatc gatttggcaa tcgcttatga agtcgccagc     180

gtttgtatct tgccctacta cttgaaacgt tgtgctgcga agctcgcggg caccggcgtg     240

aaagcgtcaa ccacgatcgg ttttcctcat ggtggtcaca ccaccgcgat caagaaagcc     300

gaagccgaac aagccatcca agatggctgc gaagaactcg acttcgtcgt caacatctcg     360

caagtcctga gcggcggttg ggactacgtc caaaatgaaa ttggcgaggt caccgaactg     420

acccatgcgg ccggacaaaa gatcaaggtg atcttcgaga actgctacct gcaggacgaa     480

cacaagattc gtctgtgcga gatctgcacc gagctcaaag tggactgggt caaaacatcg     540

actggttatg gaactggagg cgcgaccatg gacgacctgc gtctgatgcg acaacactca     600

ggcgaaaacg tccaagtcaa agctgccggt ggcgtccgag atctcgcgac actgctggag     660

gtccgagccc tcggagcatc ccgttgcggt gccagccgaa ccgccgagat gctgggcgaa     720

gcccgaaagc aacttggcat gcccgcgatt gaaatcaccg cgaccggcag ctccggctac     780

tga                                                                   783

<210>  47
<211>  260
<212>  PRT
<213>  Rhodopirellula baltica

<400>  47

Met Arg Asp Thr Arg Pro Pro Ala Ala Ser Leu Ser Leu His Gly Asn
1               5                   10                  15

Leu Leu Thr Met Ala Asp Tyr Gln Tyr His Asp Val Ser Lys Met Ile
            20                  25                  30

Asp His Ser Leu Leu Pro Pro Thr Leu Thr Glu Ala Asp Leu Asp Ser
        35                  40                  45

Gly Ile Asp Leu Ala Ile Ala Tyr Glu Val Ala Ser Val Cys Ile Leu
    50                  55                  60

Pro Tyr Tyr Leu Lys Arg Cys Ala Ala Lys Leu Ala Gly Thr Gly Val
65                  70                  75                  80

Lys Ala Ser Thr Thr Ile Gly Phe Pro His Gly Gly His Thr Thr Ala
                85                  90                  95

Ile Lys Lys Ala Glu Ala Glu Gln Ala Ile Gln Asp Gly Cys Glu Glu
            100                 105                 110

Leu Asp Phe Val Val Asn Ile Ser Gln Val Leu Ser Gly Gly Trp Asp
        115                 120                 125

Tyr Val Gln Asn Glu Ile Gly Glu Val Thr Glu Leu Thr His Ala Ala
    130                 135                 140

Gly Gln Lys Ile Lys Val Ile Phe Glu Asn Cys Tyr Leu Gln Asp Glu
145                 150                 155                 160

His Lys Ile Arg Leu Cys Glu Ile Cys Thr Glu Leu Lys Val Asp Trp
                165                 170                 175

Val Lys Thr Ser Thr Gly Tyr Gly Thr Gly Gly Ala Thr Met Asp Asp
            180                 185                 190

Leu Arg Leu Met Arg Gln His Ser Gly Glu Asn Val Gln Val Lys Ala
        195                 200                 205

Ala Gly Gly Val Arg Asp Leu Ala Thr Leu Leu Glu Val Arg Ala Leu
    210                 215                 220

Gly Ala Ser Arg Cys Gly Ala Ser Arg Thr Ala Glu Met Leu Gly Glu
225                 230                 235                 240

Ala Arg Lys Gln Leu Gly Met Pro Ala Ile Glu Ile Thr Ala Thr Gly
                245                 250                 255

Ser Ser Gly Tyr
            260

<210>  48
<211>  30
<212>  DNA
<213>  Artificial sequence

<220>
<223>  primer

<400>  48
cgggatccac tgatctgaaa gcaagcagcc                                        30

<210>  49
<211>  26
<212>  DNA
<213>  Artificial sequence

<220>
<223>  primer

<400>  49
gcaagcttgc tgctggcgct cttacc                                          26